# EUROPÄISCHE PATENTSCHRIFT

(11) **EP 1 521 595 B1**
(45) Veröffentlichungstag und Bekanntmachung des Hinweises auf die Patenterteilung: **15.03.2006**
(21) Anmeldenummer: 03762525.8
(22) Anmeldetag: 26.06.2003
(51) Int. Cl.: A61K 45/06, A61K 31/46, A61K 31/505, A61K 31/47, A61K 39/395, A61P 11/00

(54) **NEUE ARZNEIMITTELKOMPOSITIONEN AUF DER BASIS NEUER ANTICHOLINERGIKA UND EGFR-KINASE HEMMERN**
NOVEL DRUG COMPOSITIONS BASED ON NOVEL ANTICHOLINERGICS AND INHIBITORS OF EGFR-KINASE
NOUVELLES COMPOSITIONS DE MEDICAMENTS A BASE DE NOUVEAUX ANTICHOLINERGIQUES ET D'INHIBITEURS DE L'EGFR-KINASE

(30) Priorität: 09.07.2002 DE 10230751
(43) Veröffentlichungstag der Anmeldung: 13.04.2005
(60) Teilanmeldung: 05109909.1
(73) Patentinhaber: Boehringer Ingelheim Pharma GmbH & Co.KG, 55216 Ingelheim am Rhein (DE)
(72) Erfinder: PAIRET, Michel, 88400 Biberach (DE); MEADE, Christopher, John, Montague, 88437 Maselheim (DE); PIEPER, Michael, P., 88400 Biberach (DE); JUNG, Birgit, 88471 Laupheim (DE)
(86) Internationale Anmeldenummer: PCT/EP2003/006788
(87) Internationale Veröffentlichungsnummer: WO 2004/004775

(56) Entgegenhaltungen:
- WO-A-00/69468
- WO-A-02/32899
- US-A- 2 927 925

## Beschreibung

Die vorliegende Erfindung betrifft neuartige Arzneimittelkompositionen auf der Basis neuer Anticholinergika und EGFR-Kinase-Hemmern, Verfahren zu deren Herstellung sowie deren Verwendung bei der Therapie von Atemwegserkrankungen.

### Beschreibung der Erfindung

Die vorliegende Erfindung betrifft neuartige Arzneimittelkompositionen auf der Basis neuer Anticholinergika und EGFR-Kinase-Hemmem, Verfahren zu deren Herstellung sowie deren Verwendung bei der Therapie von Atemwegserkrankungen.

Überraschenderweise kann ein unerwartet vorteilhafter therapeutischer Effekt, insbesondere ein synergistischer Effekt bei der Behandlung von entzündlichen und/oder obstruktiven Atemwegserkrankungen beobachtet werden, wenn ein oder mehrere, bevorzugt ein Anticholinergikum der Formel **1** gemeinsam mit einem oder mehreren, bevorzugt einem EGFR-Kinase-Hemmer **2** zur Anwendung gelangen. Aufgrund dieses synergistischen Effekts sind die erfindungsgemäßen Arzneimittelkombinationen unter geringerer Dosierung einsetzbar, als dies bei der sonst üblichen Monotherapie der Einzelverbindungen der Fall ist.

Die erfindungsgemäßen Wirkstoffkombinationen sind überraschenderweise ferner sowohl durch einen raschen Wirküngseintritt, als auch durch eine langandauernde Wirkdauer gekennzeichnet. Dies ist von hoher Bedeutung für das Wohlbefinden des Patienten, da er einerseits nach Applikation der Kombination eine rasche Verbesserung seines Zustands verspürt und andererseits aufgrund der langen Wirkdauer eine einmal pro Tag erfolgende Applikation ausreichend ist.
Die vorstehend genannten Effekte werden sowohl bei gleichzeitiger Applikation innerhalb einer einzigen Wirkstoffformulierung als auch bei sukzessiver Applikation der beiden Wirkstoffe in getrennten Formulierungen beobachtet. Erfindungsgemäß bevorzugt ist die gleichzeitige Applikation der beiden Wirkstoffbestandteile in einer einzigen Formulierung.

Im Rahmen der vorliegenden Erfindung gelangen als Anticholinergikum die Salze der Formel **1** 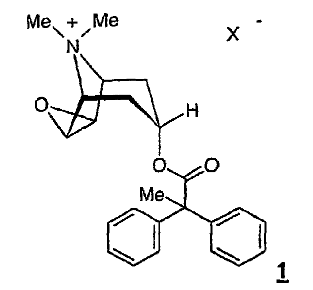
worin
- X⁻: ein einfach negativ geladenes Anion, vorzugsweise ein Anion ausgewählt aus der Gruppe bestehend aus Chlorid, Bromid, lodid, Sulfat, Phosphat, Methansulfonat, Nitrat, Maleat, Acetat, Citrat, Fumarat, Tartrat, Oxalat, Succinat, Benzoat und p- Toluolsulfonat bedeutet zur Anwendung.

Bevorzugt gelangen die Salze der Formel **1** zur Anwendung, worin
- X⁻: ein einfach negativ geladenes Anion ausgewählt aus der Gruppe Chlorid, Bromid, 4-Toluolsulfonat und Methansulfonat, bevorzugt Bromid, bedeutet.

Besonders bevorzugt gelangen die Salze der Formel **1** zur Anwendung, worin
- X⁻: ein einfach negativ geladenes Anion ausgewählt aus der Gruppe Chlorid, Bromid und Methansulfonat, bevorzugt Bromid, bedeutet.

Erfindungsgemäß besonders bevorzugt ist dasjenige Salz der Formel **1,** in dem X⁻ für Bromid steht.

Die Salze der Fromel **1** sind aus der Internationalen Patentanmeldung WO 02/32899 bekannt.

Im Rahmen der vorliegenden Patentanmeldung ist eine explizite Bezugnahme auf das pharmakologisch aktive Kation der Formel 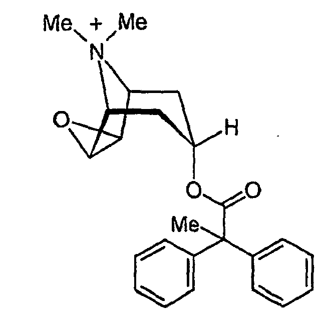 durch Verwendung der Bezeichnung **1'** zu erkennen. Eine Bezugnahme auf Verbindungen **1** schließt naturgemäß eine Bezugnahme auf das Kation **1'** mit ein.

Im Rahmen der vorliegenden Erfindung werden unter EGFR-Kinase-Hemmern (im Folgenden **2)** vorzugsweise solche Verbindungen verstanden, die ausgewählt sind aus der Gruppe bestehend aus 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((*S*)-6-methyl-2-oxo-morpholin-4-yl)-ethoxy]-6-[(vinylcarbonyl)amino]-chinazolin, 4-[(3-Chlor-4-fluor-phenyl)amino]-7-[4-((*R*)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino]-7-cyclopropylmethoxy-chinazolin, 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(*R*)-(1 -Phenyl-ethyl)amino]-6-{[4-(morpholin-4-yl)-1 -oxo-2-buten-1-yl]-amino}-7-cyclopropylmethoxy-chinazolin, 4-[(*R*)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazolin, 4-[(3-Chlor-4-fluor-phenyl)amino]-6-{[4-{(*R*)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((*S*)-6-methyl-2-oxo-marpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(*R*)-(1-Phenyl-ethyl)amino]-6-({4-[N-(2-methoxy-ethyl)-N-ethyl-amino]-1-oxo-2-buten-1-yl)amino)-7-cyclopropylmethoxy-chinazolin, 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(*R*)-(1-Phenylethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6{[4-(N,N-dimethylamino)-1-oxo-2-buten-1-yl]aminol-7-[(*R*)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin, 4-[(3-Chlor-4-fluorphenyl)amino]-6-[3-(morpholin-4-yl)-propyloxy]-7-methoxy-chinazolin, 4-[(3-Ethinyl-phenyl)amino]-6,7-bis-(2-methoxy-ethoxy)-chinazolin, 4-[(3-Chlor-4-fluorphenyl)amino]-7-(3-(morpholin-4-yl)-propyloxy]-6-[(vinylcarbonyl)amino]-chinazolin, 4-[(*R*)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, Cetuximab, Trastuzumab, ABX-EGF und Mab ICR-62.

Bevorzugt sind die EGFR-Kinase-Hemmer **2** ausgewählt aus der Gruppe bestehend aus 4-[(3-Chlor-4-fluor-phenyl)amino]-7-(2-{4-[(S)-(2-oxo-tetrahydrofuran-5-yl)-carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, 4-[(3-Chlor-4-fluor-phenyl)amino]-7-[4-(2,2-dimethyl-6-oxo-morpholin-4-yl)-butyloxy]-6-[(vinylcarbonyl)amino]-chinazolin, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{(4-(N,N-diethylamino)-1-oxo-2-buten-1-y]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(3-Chlor-4-fluorphenyl)amino]-6-[(4-{N-[2-(ethoxycarbonyl)-ethyl]-N-[(ethoxycarbonyl)methyl]amino)-1-oxo-2-buten-1-yl)amino]-7-cyclopropylmethoxy-chinazolin, 4-[(R)-(1-Phenyl-ethyl)amino]-6-{[4-(morpholin-4-yl)-1-oxo-2-buten-1-yl]aminol-7-cyclopropylmethoxy-chinazolin, 4-[(R)-(1-Phenylethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-([4-((R)-6-methyl-2-oxo-morpholin-4-yl)-1-oxo-2-buten-1-yl]amino)-7-cyclopropylmethoxy-chinazolin, 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin, 4-[(3-Chlor-4-fluor-phenyl)amino)-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-{(R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6{[4-(N,N-dimethylamino)-1-oxo-2-buten-1-yl]amino}-7-cyclopentyloxy-chinazolin, 4-[(3-Chlor-4-fluor-phenyl)amino]-6-{[4-((S)-2-methoxymethyl-6-oxo-morpholin-4-yl)-1-oxo-2-buten-1-yl]amino}-7-cyclopropylmethoxy-chinazolin, 4-[(R)-(1 -Phenyl-ethyl)amino]-6-([4-(N, N-bis-(2-methoxy-ethyl)-amino)-1-oxo-2-buten-1-yl]amino)-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethytamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino)-7-((S)-tetrahydrofuran-3-yloxy)-chinazolin, 4-[(3-Chlor-4-fluorphenyl)amino]-6-({4-[N-(2-methoxy-ethyl)-N-methy)-amino]-1-oxo-2-buten-1 - yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]aminol-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino)-7-[(S)-(tetrahydrofuran-2-yl)methoxy]-chinazolin, 4-[(3-Chlor-4-fluor-phenyl)amino]-6-[(4-dimethylamino-cyclohexyl)amino]-pyrimido[5,4-d]pyrimidin oder 4-[(3-Chlor-4-fluorphenyl)amino]-6-[3-(morpholin-4-yl)-propyloxy]-7-methoxy-chinazolin.

Besonders bevorzugt sind die EGFR-Kinhase-Hemmer **2** ausgewählt aus der Gruppe bestehend aus 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butytoxy]-6-[(vinylcarbonyl)amino]-chinazolin, 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopropylmethoxy-chinazolin und 4-[(3-Chlor-4-fluorphenyl)amino]-6-[3-(morpholin-4-yl)-propyloxy]-7-methoxy-chinazolin.

Eine Bezugnahme auf die vorstehend genannten EGFR-Kinase-Hemmer **2** schließt im Rahmen der vorliegenden Erfindung eine Bezugnahme auf deren gegebenenfalls existierende pharmakologisch verträgliche Säureadditionssalze ein.

Unter den physiologischbzw. pharmakologisch verträglichen Säureadditionssalzen, die von **2** gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind die Salze der Verbindungen **2** solche, die ausgewählt sind aus den Salzen der Essigsäure, Salzsäure, Bromwasserstoffsäure, Schwefelsäure, Phosphorsäure uns Methansulfonsäure.

Die Applikation der erfindungsgemäßen Arzneimittelkombinationen aus **1** und **2** erfolgt.vorzugsweise auf inhalativem Wege. Hierbei können geeignete Inhaltionspulver, die in geeignete Kapseln (inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten, Die inhalative Applikation kann ferner mittels geeigneter Lösungen der Arzneimittelkombination bestehend aus **1** und **2** erfolgen.

Ein Aspekt der vorliegenden Erfindung betrifft dementsprechend ein Arzneimittel, welches eine Kombination aus **1** und **2** enthält.

Ein weiterer Aspekt der vorliegenden Erfindung betrifft ein Arzneimittel, welches ein oder mehrere Salze 1 und ein oder mehrere Verbindungen 2, gegebenfalls in Form ihrer Solvate oder Hydrate enthält. Auch hierbei können die Wirkstoffe entweder gemeinsam in einer einzigen Darreichungsform oder in zwei getrennten Darreichungsformen enthalten sein. Erfindungsgemäß bevorzugt sind Arzneimittel, die die Wirkstoffe **1** und **2** in einer einzigen Darreichungsform enthalten.

Ein weiterer Aspekt der vorliegenden Erfindung betrifft ein Arzneimittel, welches neben therapeutisch wirksamen Mengen von **1** und **2** einen pharmazeutisch verträglichen Trägerstoff bzw. Hilfsstoff enthält. Ein weiterer, besonders bevorzugter Aspekt der vorliegenden Erfindung betrifft ein Arzneimittel, welches neben therapeutisch wirksamen Mengen von 1 und 2 keinen pharmazeutisch verträglichen Trägerstoff bzw. Hilfsstoff enthält.

Die vorliegende Erfindung betrifft ferner die Verwendung von **1** und **2** zur Herstellung eines therapeutisch wirksame Mengen von 1 und 2 enthaltenden Arzneimittels zur Behandlung von entzündlichen oder obstruktiven Atemwegserkrankungen, insbesondere von Asthma oder chronisch obstruktiven Lungenerkrankungen (COPD), sowie deren Komplikationen wie beispielsweise pulmonale Hypertension, daneben auch allergische und nicht allergische Rhinitis, sofern eine Behandlung mit EGFR-Kinase-Hemmer aus therapeutischer Sicht nicht kontraindiziert ist, durch simultane oder sukzessive Applikation.

Die vorliegende Erfindung zielt ferner auf die simultane oder sukzessive Verwendung therapeutisch wirksamer Dosen der Kombination vorstehender Arzneimittel **1** und **2** zur Behandlung von entzündlichen und/oder obstruktiven Atemwegserkrankungen, insbesondere von Asthma oder chronisch obstruktiver Lungenerkrankung (COPD), sowie deren Komplikationen wie beispielsweise pulmonale Hypertension, daneben auch allergische und nicht allergische Rhinitis, sofern eine Behandlung mit EGFR-Kinase-Hemmern aus therapeutischer Sicht nicht kontraindiziert ist, durch simultane oder sukzessive Applikation.

In den erfindungsgemäßen Wirkstoffkombinationen aus 1 und 2 können die Bestandteile 1 und 2 in Form ihrer Enantiomere, Gemische der Enantiomere oder in Form der Racemate enthalten sein.

Die Verhältnisse, in denen die beiden Wirkstoffe **1** und **2** in die erfindungsgemäßen Wirkstoffkombinationen eingesetzt werden können, sind variabel. Die Wirkstoffe **1** und 2 können gegebenfalls in Form ihrer Solvate oder Hydrate vorliegen. Je nach Wahl der Verbindungen **1** bzw. **2** variieren die im Rahmen der vorliegenden Erfindung einsetzbaren Gewichtsverhältnisse aufgrund des unterschiedlichen Molekulargewichts der verschiedenen Verbindungen sowie aufgrund ihrer unterschiedlichen Wirkstärke. In der Regel können die erfindungsgemäßen Arzneimittelkombinationen die Verbindungen **1** und **2** in Gewichtsverhältnissen enthalten, die in einem Bereich von 1:300 bis 60:1, bevorzugt von 1:200 bis 30:1, liegen. Bei den besonders bevorzugten Arzneimittelkombinationen, die neben einer Verbindung der Formel **1** eine Verbindung ausgewählt aus der Gruppe bestehend aus 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, 4-[(3-Chlor-4-fluor-phenyl)amino]-7-(2-{4-[(S)-(2-oxo-tetrahydrofuran-5-yl)-carbonyl]-piperazin-1-yl}-ethoxy)-6-[(vinyicarbonyl)amino]-chinazolin, 4-[(3-Chlor-4-fluor-phenyl)amino]-7-[2-((*S*)-6-methyl-2-oxo-morpholin-4-yl)-ethoxy]-6-[(vinylcarbonyl)amino]-chinazolin, 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(R)-(1-Phenyl-ethyl)amino]-6-{(4-(morpholin-4-yl)-1-oxo-2-buten-1-yl]amino}-7-cyclopropylmethoxy-chinazolin und 4-[(3-Chlor-4-fluorphenyl)amino]-6-[3-(morpholin-4-yl)-propyloxy]-7-methoxy-chinazolin als **EGFR-Kinase-Inhibitoren 2** enthalten, liegen die Gewichtsverhältnisse von **1** zu **2** besonders bevorzugt in einem Bereich, in dem das Kation **1'** und **2** in Verhältnissen von 1:180 bis 15:1, ferner bevorzugt von 1:150 bis 3:1, besonders bevorzugt von 1:100 bis 1:30 enthalten sind.

Die Anwendung der erfindungsgemäßen Arzneimittel enthaltend die Kombinationen aus **1** und **2** erfolgt üblicherweise so, daß 1 und 2 gemeinsam in Dosierungen von 1000 bis 100000*µ*g, bevorzugt von 1500 bis 50000*µ*g, besonders bevorzugt von 2000 bis 10000*µ*g, ferner bevorzugt von 2500 bis 7500*µ*g pro Einmalgabe enthalten sind. Beispielsweise enthalten erfindungsgemäße Kombinationen aus **1** und **2** eine solche Menge an **1'** und EGFR-Kinase-Hemmer 2, daß die Gesamtdosierung pro Einmalgabe 2500*µ*g, 2550*µ*g, 2600*µ*g, 2650*µ*g, 2700*µ*g, 2750*µ*g, 2800*µ*g, 2850*µ*g, 2900*µ*g, 2950*µ*g, 3000*µ*g, 3050*µ*g, 3100*µ*g, 3150*µ*g, 3200*µ*g, 3250*µ*g, 3300*µ*g, 3350*µ*g, 3400*µ*g, 3450*µ*g, 3500*µ*g, 3550*µ*g, 3600*µ*g, 3650*µ*g, 3700*µ*g, 3750*µ*g, 3800*µ*g, 3850*µ*g, 3900*µ*g, 3950*µ*g, 4000*µ*g, 4050*µ*g, 4100*µ*g, 4150*µ*g, 4200*µ*g, 4250*µ*g, 4300*µ*g, 4350*µ*g, 4400*µ*g, 4450*µ*g, 4500*µ*g, 4550*µ*g, 4600*µ*g, 4650*µ*g, 4700*µ*g, 4750*µ*g, 4800*µ*g, 4850*µ*g, 4900*µ*g, 4950*µ*g, 5000*µ*g, 5050*µ*g, 5100*µ*g, 5150*µ*g, 5200*µ*g, 5250*µ*g, 5300*µ*g, 5350*µ*g, 5400*µ*g, 5450*µ*g, 5500*µ*g, 5550*µ*g, 5600*µ*g, 5650*µ*g, 5700*µ*g, 5750*µ*g, 5800*µ*g, 5850*µ*g, 5900*µ*g, 5950*µ*g, 6000*µ*g, 6050*µ*g, 6100*µ*g, 6150*µ*g, 6200*µ*g, 6250*µ*g, 6300*µ*g, 6350*µ*g, 6400*µ*g, 6450*µ*g, 6500*µ*g, 6550*µ*g, 6600*µ*g, 6650*µ*g, 6700*µ*g, 6750*µ*g, 6800*µ*g, 6850*µ*g, 6900*µ*g, 6950*µ*g, 7000*µ*g, 7050*µ*g, 7100*µ*g, 7150*µ*g, 7200*µ*g, 7250*µ*g, 7300*µ*g, 7350*µ*g, 7400*µ*g, 7450*µ*g, 7500*µ*g oder ähnliches beträgt. Vorstehend genannte Dosierungsvorschläge pro Einmalgabe sind nicht als auf die explizit angegebenen Zahlenwerte beschränkt anzusehen, sondern dienen nur als beispielhaft offenbarte Dosierungen. Selbstverständlich sind beispielsweise auch Dosierungen, die um o.g. Zahlenwerte in einem Bereich von ca. +/- 25*µ*g schwanken, von den vorliegenden exemplarisch erläuterten Werten umfaßt. Bei diesen Dosierungsbereichen können die Wirkstoffe 1' und 2 in den vorhergehend beschriebenen Gewichtsverhältnissen enthalten sein.

Beispielsweise und ohne den Umfang der Erfindung darauf zu beschränken, können die erfindungsgemäßen Kombinationen aus **1** und **2** eine solche Menge an **1**' und EGFR-Kinase-Hemmer **2** enthalten, daß pro Einmalgabe 16,5*µ*g **1'** und 2500*µ*g **2**, 16,5*µ*g **1**' und 3000*µ*g **2,** 16,5*µ*g **1**' und 3500*µ*g **2**, 16,5*µ*g **1**' und 4000*µ*g **2,** 16,5*µ*g **1'** und 4500*µ*g **2**, 16,5*µ*g **1**' und 5000*µ*g **2**, 16,5*µ*g **1**' und 5500*µ*g **2**, 16,5*µ*g **1'** und 6000*µ*g **2,** 16,5*µ*g **1'** und 6500*µ*g **2,** 16,5*µ*g **1'** und 7000*µ*g **2**, 33,1*µ*g **1**' und 2500*µ*g **2**, 33,1*µ*g **1'** und 3000*µ*g **2,** 33,1*µ*g **1'** und 3500*µ*g **2**, 33,1*µ*g **1'** und 4000*µ*g **2**, 33,1*µ*g **1**' und 4500*µ*g **2**, 33,1*µ*g **1**' und 5000*µ*g **2**, 33,1*µ*g **1'** und 5500*µ*g **2,** 33,1*µ*g **1'** und 6000*µ*g **2**, 33,1*µ*g **1'** und 6500*µ*g **2,** 33,1*µ*g **1**' und 7000*µ*g **2**, 49,5*µ*g **1**' und 2500*µ*g **2**, 49,5*µ*g **1'** und 3000*µ*g **2**, 49,5*µ*g **1**' und 3500*µ*g **2**, 49,5*µ*g **1**' und 4000*µ*g **2**, 49,5*µ*g **1**' und 4500*µ*g **2**, 49,5*µ*g **1'** und 5000*µ*g **2**, 49,5*µ*g **1'** und 5500*µ*g **2**, 49,5*µ*g **1**' und 6000*µ*g 2, 49,5*µ*g **1**' und 6500*µ*g **2**, 49,5*µ*g 1' und 7000*µ*g **2,** 82,6*µ*g **1'** und 2500*µ*g **2**, 82,6*µ*g **1**' und 3000*µ*g **2,** 82,6*µ*g **1'** und 3500*µ*g **2,** 82,6*µ*g **1'** und 4000*µ*g **2,** 82,6*µ*g **1' und** 4500*µ*g **2**, 82,6*µ*g **1**' und 5000*µ*g **2,** 82,6*µ*g **1'** und 5500*µ*g **2,** 82,6*µ*g **1'** und 6000*µ*g **2,** 82,6µg **1** ' und 6500*µ*g 2, 82,6*µ*g **1'** und 7000*µ*g **2,** 165, 1*µ*g **1'** und 2500*µ*g **2,** 165,1*µ*g **1'** und 3000*µ*g **2, 165,1*µ*g 1'** und 3500*µ*g **2,** 165,1*µ*g **1'** und 4000*µ*g **2,** 165,1*µ*g **1'** und 4500*µ*g **2,** 165,1*µ*g **1'** und 5000*µ*g **2**, 165,1*µ*g **1'** und 5500*µ*g **2,** 165,1*µ*g **1'** und 6000*µ*g **2**, 165,1*µ*g **1'** und 6500*µ*g **2**, 165,1*µ*g **1'** und 7000*µ*g **2**, 206,4*µ*g **1'** und 2500*µ*g **2**, 206,4*µ*g **1'** und 3000*µ*g **2**, 206,4*µ*g **1'** und 3500*µ*g **2,** 206,4*µ*g **1'** und 4000*µ*g **2**, 206,4*µ*g **1'** und 4500*µ*g **2**, 206,4*µ*g **1'** und 5000*µ*g **2,** 206,4*µ*g **1'** und 5500*µ*g **2**, 206,4*µ*g **1'** und 6000*µ*g **2**, 206,4*µ*g **1**' und 6500*µ*g **2**, 206,4*µ*g **1'** und 7000*µ*g **2**, 412,8*µ*g **1'** und 2500*µ*g **2**, 412,8*µ*g **1**' und 3000*µ*g **2**, 412,8*µ*g **1**' und 3500*µ*g **2**, 412,8*µ*g **1'** und 4000*µ*g **2**, 412,8*µ*g **1'** und 4500*µ*g **2,** 412,8*µ*g **1**' und 5000*µ*g **2**, 412,8*µ*g **1**' und 5500*µ*g **2**, 412,8*µ*g **1**' und 6000*µ*g **2**, 412,8*µ*g **1**' und 6500*µ*g **2** oder 412,8*µ*g **1**' und 7000*µ*g **2** appliziert werden.

Wird als erfindungsgemäß bevorzugte Kombination aus **1** und **2** die Wirkstoffkombination verwendet, in der **1** für das Bromid steht, entsprechen die vorstehend beispielhaft genannten pro Einmalgabe applizierten Wirkstoffmengen von **1'** und **2** den nachfolgenden pro Einmalgabe applizierten Mengen an **1** und **2:** 20*µ*g **1** und 2500*µ*g **2,** 20*µ*g **1** und 3000*µ*g **2,** 20*µ*g 1 und 3500*µ*g 2, 20*µ*g **1** und 4000*µ*g **2**, 20*µ*g **1** und 4500*µ*g **2,** 20*µ*g **1** und 5000*µ*g **2,** 20*µ*g **1** und 5500*µ*g **2,** 20*µ*g **1** und 6000*µ*g **2,** 20*µ*g **1** und 6500µg **2**, 20*µ*g **1** und 7000*µ*g **2,** 40*µ*g **1** und 2500*µ*g **2,** 40*µ*g **1** und 3000*µ*g **2**, 40*µ*g **1** und 3500*µ*g **2**, 40*µ*g 1 und 4000*µ*g **2,** 40*µ*g **1** und 4500*µ*g **2**, 40*µ*g **1** und 5000*µ*g **2**, 40*µ*g 1 und 5500*µ*g **2,** 40*µ*g **1** und 6000*µ*g **2**, 40*µ*g **1** und 6500*µ*g **2**, 40*µ*g **1** und 7000*µ*g **2,** 60*µ*g **1** und 2500*µ*g **2**, 60*µ*g **1** und 3000*µ*g **2**, 60*µ*g **1** und 3500*µ*g **2**, 60*µ*g **1** und 4000*µ*g **2**, 60*µ*g **1** und 4500*µ*g **2**, 60*µ*g **1** und 5000*µ*g **2**, 60*µ*g **1** und 5500*µ*g **2**, 60*µ*g **1** und 6000*µ*g **2**, 60*µ*g **1** und 6500*µ*g **2**, 60*µ*g **1** und 7000*µ*g **2**, 100*µ*g **1** und 2500*µ*g **2**, 100*µ*g **1** und 3000*µ*g **2**, 100*µ*g **1** und 3500*µ*g **2**, 100*µ*g **1** und 4000*µ*g **2**, 100*µ*g **1** und 4500*µ*g **2**, 100*µ*g **1** und 5000*µ*g **2**, 100*µ*g **1** und 5500*µ*g **2**, 100*µ*g **1** und 6000*µ*g **2**, 100*µ*g **1** und 6500*µ*g **2**,100*µ*g **1** und 7000*µ*g **2**, 200*µ*g **1** und 2500*µ*g **2**, 200*µ*g **1** und 3000*µ*g **2**, 200*µ*g **1** und 3500*µ*g **2**, 200*µ*g **1** und 4000*µ*g **2**, 200*µ*g **1** und 4500*µ*g **2**, 200*µ*g **1** und 5000*µ*g **2**, 200*µ*g 1 und 5500*µ*g **2**, 200*µ*g **1** und 6000*µ*g **2**, 200*µ*g **1** und 6500*µ*g **2**, 200*µ*g **1** und 7000*µ*g **2**, 250*µ*g **1** und 2500*µ*g **2***,* 250*µ*g **1** und 3000*µ*g **2**, 250*µ*g **1** und 3500*µ*g **2**, 250*µ*g **1** und 4000*µ*g **2**, 250*µ*g **1** und 4500*µ*g **2**, 250*µ*g **1** und 5000*µ*g **2**, 250*µ*g **1** und 5500*µ*g **2**, 250*µ*g **1** und 6000*µ*g **2**, 250*µ*g **1** und 6500*µ*g **2** oder 250*µ*g **1** und 7000*µ*g 2. 500*µ*g **1** und 2500*µ*g **2**, 500*µ*g **1** und 3000*µ*g **2**, 500*µ*g **1** und 3500*µ*g **2**, 500*µ*g **1** und 4000*µ*g **2**, 500*µ*g **1** und 4500*µ*g **2**, 500*µ*g **1** und 5000*µ*g **2**, 500*µ*g **1** und 5500*µ*g **2**, 500*µ*g **1** und 6000*µ*g **2**, 500*µ*g **1** und 6500*µ*g **2** oder 500*µ*g **1** und 7000*µ*g **2**.

Die Applikation der erfindungsgemäßen Wirkstoffkombinationen aus **1** und **2** erfolgt bevorzugt auf inhalativem Wege. Hierzu müssen die Bestandteile **1** und **2** in inhatierbaren Darreichungsformen bereitgestellt werden. Als inhalierbare Darreichungsformen kommen Inhalationspulver, treibgashaltige Dosieraerosole oder treibgasfreie Inhalationslösungen in Betracht. Erfindungsgemäße lnhalationspulver enthaltend die Wirkstoffkombination aus **1** und **2** können allein aus den genannten Wirkstoffen oder aus einem Gemisch der genannten Wirkstoffe mit physiologisch verträglichen Hilfsstoffen bestehen. Im Rahmen der vorliegenden Erfindung sind von dem Begriff treibgasfreie Inhaltionslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

### A) Inhalationspulver enthattend die erfindungsgemäßen Wirkstoffkombinationen aus 1 und 2:

Die erfindungsgemäßen Inhaltionspulver können **1** und **2 entweder** allein oder im Gemisch mit geeigneten physiologisch unbedenkliche Hilfsstoffen enthalten.

Sind die Wirkstoffe **1** und **2** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250*µ*m, bevorzugt zwischen 10 und 150*µ*m, besonders bevorzugt zwischen 15 und 80*µ*m auf. Gegebenenfalls kann es sinnvoll erscheinen, den vorstehend genannten Hilfststoffen feinere Hilfsstofffraktionen mit einer mittleren Teilchengröße von 1 bis 9*µ*m beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1** und **2,** vorzugsweise mit einer mittleren Teilchengröße von 0,5 bis 10*µ*m, besonders bevorzugt von 1 bis 6*µ*m, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhaltionspulver können entweder in Form einer einzigen Pulvermischung, die **sowohl 1** als auch **2** enthält oder in Form von separaten Inhalationspulvern, die lediglich **1** und 2 enthalten bereitgestellt und appliziert werden.

Die erfindungsgemäßen Inhalationspulver können mittels aus dem Stand der Technik bekannten Inhalatoren appliziert werden. Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2** physiologisch unbedenkliche Hilfsstoff enthalten, allerdings in Kapseln abgefüllt (zu sogenannten Inhaletten), die in Inhalatoren wie beispielsweise in der WO 94/28958 beschrieben, zur Anwendung gelangen.

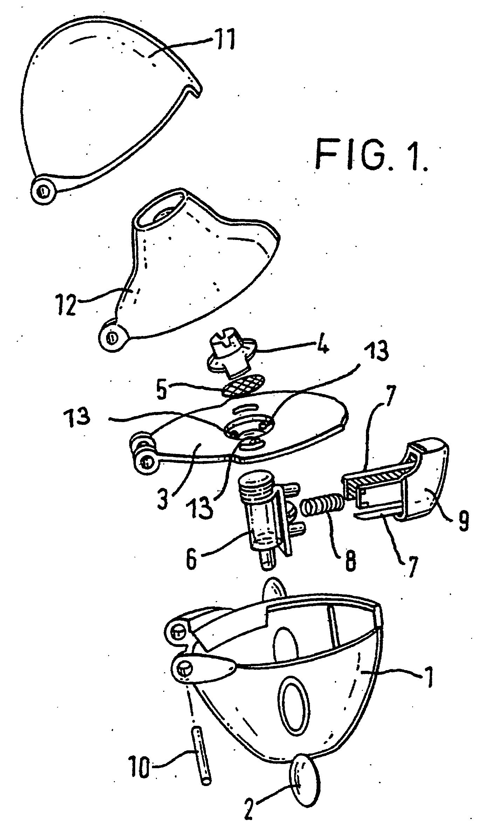

Ein zur Anwendung der erfindungsgemäßen Arzneimittelkombination in Inhaletten besonders bevorzugter Inhalator ist Figur 1 zu entnehmen.
Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 8 vorgesehen ist, sowie ein über eine Achse 10 klappbar mit dem Gehäuse 1, dem Deck 3 und einer Kappe 11 verbundenes Mundstück 12 sowie Luftdurchlasslöcher 13 zur Einstellung des Strömungswiderstands.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 50mg, bevorzugt von 3 bis 45mg, bevorzugt 5 bis 40 mg Inhalationspulver pro Kapsel an. Diese enthalten erfindungsgemäß entweder gemeinsam oder jeweils die bereits vorstehend für **1'** und **2** genannten Dosierungen pro Einmalgabe.

### B) Treibgashaltige Inhalationsaerosole enthaltend die erfindungsgemäßen Wirkstoffkombinationen aus 1 und 2:

Erfindungsgemäße treibgashaltige Inhaltionsaerosole können **1** und **2** im Treibgas gelöst oder in dispergierter Form enthalten. Hierbei können **1** und **2** in getrennten Darreichungsformen oder in einer gemeinsamen Darreichungsform enthalten sein, wobei **1** und **2** entweder beide gelöst, beide dispergiert oder jeweils nur eine Komponente gelöst und die andere dispergiert enthalten sein können.

Die zur Herstellung der erfindungsgemäßen Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder isobutan und Halogenkohlenwasserstoffen wie chlorierten und/oder fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus der Gruppe bestehend aus TG11, TG12, TG134a und TG227. Von den vorstehend genannten halogenierten Kohlenwasserstoffen sind erfindungsgemäß das TG134a (1,1,1,2-Tetrafluorethan) und das TG227 (1,1,1,2,3,3,3-Heptafluorpropan) und Mischungen derselben bevorzugt.

Die erfindungsgemäßem treibgashaltigen Inhalationsaerosole können femer weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhaltionsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2.**

Liegen die Wirkstoffe **1** und/oder **2** in dispergierter Form vor weisen die Wirkstoffteilchen bevorzugt eine mittlere Teilchengröße von bis zu 10 *µ*m, bevorzugt von 0,1 bis 5 *µ*m, besonders bevorzugt von 1 bis 5 *µ*m auf.

Die vorstehend genannten erfindungsgemäßen treibgashaltigen Inhaltionaerosole können mittels im Stand der Technik bekannten Inhalatoren (MDls = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, daß sie vorstehend beschriebene erfindungsgemäße treibgashaltige Aerosole enthalten.
Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

### C) Treibgasfreie Inhaltionslösungen oder Suspensionen enthaltend die erfindungsgemäßen Wirkstoffkombinationen aus 1 und 2:

Besonders bevorzugt erfolgt die Applikation der erfindungsgemäßen Wirkstoffkombination in Form von treibgasfreien Inhalationslösungen und Inhaltionssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** und **2 ,** getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg /100 ml, bevorzugt unter 50 mg/100 ml, besonders beovorzugt unter 20 mg/100 ml.

Generell sind solche Inhalüonslösungen bevorzugt, in denen der Gehalt an Natriumedetat bei 0 bis 10mg/100ml liegt.

Den erfindungsgemäßen treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere lsopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu denZusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100*µ*L, bevorzugt weniger als 50*µ*L, besonders bevorzugt zwischen 20 und 30*µ*L Wirkstofflösung mit bevorzugt einem Hub zu einem Aerosol mit einer durchschnittlichen Teilchengröße von weniger als 20 *µ*m, bevorzugt weniger als 10*µ*m, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat® bekannt.

Dieser Vernebler (Respimat®) kann vorteilhaft zur Erzeugung der erfindungsgemäßen inhalierbaren Aerosole enthaltend die Wirkstoffkombination aus 1 und 2 eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4-insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahtrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.

Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687, auf die an dieser Stelle explizit Bezug genommen wird, ist der Vernebler (Respimat®) beschrieben, mit dem die erfindungsgemäßen wäßrigen Aerosolzubereitungen vorteilhaft inhaliert werden können. Figur 6a der WO 97/12687 zeigt einen Längsschnitt durch den Zerstäuber bei gespannter Feder, Figur 6b der WO 97/12687 zeigt einen Längsschnitt durch den Zerstäuber bei entspannter Feder. Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt.

Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Dementsprechend betrifft ein weiterer Aspekt der vorliegenden Erfindung Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat® Bevorzugt zielt die vorliegende Erfindung auf treibgasfreie Inhaltionslösungen oder Suspensionen gekennzeichnet durch die erfindungsgemäßen Wirkstoffkombination aus 1 und 2 in Verbindung mit der unter der Bezeichnung Respimat® bekannten Vorrichtung. Ferner betrifft die vorliegende Erfindung vorstehend genannte Vorrichtungen zur Inhalation, bevorzugt den Respimat®, dadurch gekennzeichnet, daß sie vorstehend beschriebene erfindungsgemäße treibgasfreie Inhaltionslösungen oder Suspensionen enthalten.

Die erfindungsgemäßen treibgasfreien Inhalationslösungen oder Suspensionen können neben den vorstehend, zur Applikation im Respimat vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, daß es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Ausganqsmaterialien

Zur Darstellung der im Rahmen der vorliegenden Erfindung genannten und noch nicht im Stand der Technik bekannten Verbindungen **2:**

### I.) 4-[(3-Chlor-4-fluor-phenyl)amino]-7-{3-[4-(2-oxo-tetrahydrofuran-4-yl)-piperazin-1-yl]-propyloxy}-6-[(vinylcarbonyl)amino]-chinazolin

Eine Mischung aus 166 mg Acrylsäure und 0.77 ml Triethylamin in 10 ml Tetrahydrofuran wird im Trockeneis/Aceton-Kühlbad auf -50°C abgekühlt und mit einer Lösung aus 175 *µ*l Acrylsäurechlorid in 4 ml Tetrahydrofuran versetzt. Das Reaktionsgemisch wird 45 Minuten bei dieser Temperatur gerührt. Anschließend wird eine Lösung aus 427 mg 6-Amino-4-[(3-chlor-4-fluor-phenyl)amino]-7-{3-[4-(2-oxo-tetrahydrofuran-4-yl)-piperazin-1-yl]-propyloxy}-chinazolin in 10 ml Tetrahydrofuran innerhalb von 20 Minuten zugetropft. Nun läßt man das Reaktionsgemisch langsam auf 0°C erwärmen und rührt bei dieser Temperatur, bis die Umsetzung vollständig ist. Anschließend wird mit Eiswasser versetzt, wobei sich ein zäher Niederschlag bildet. Dieser wird mehrmals gründlich mit Essigester/Methanol extrahiert. Die vereinigten organischen Phasen werden mit gesättigter Natriumchlorid-Lösung gewaschen, über Magnesiumsulfat getrocknet und eingeengt. Das gelbliche, harzartige Rohprodukt wird chromatographisch über eine Kieselgelsäule mit Methylenchlorid/Methanol (95:5) als Laufmittel gereinigt.
Ausbeute: 148 mg (31 % der Theorie),
R_{f}-Wert: 0.45 (Kieselgel, Methylenchlorid/Methanol/konzentrierte, wäßrige Ammoniaklösung = 90:10:0.1)
Massenspektrum (ESI⁺): m/z = 567, 569 [M-H]⁺

Analog zu 1.) wird folgende Verbindung erhalten:

4-[(3-Chlor-4-fluor-phenyl)amino]-7-(2-{4-[(*S*)-(2-oxo-tetrahydrofuran-5-yl)carbonyl]-piperazin-1-yl}-ethoxy)-6-[(vinylcarbonyl)amino]-chinazolin
R_{f}-Wert: 0.46 (Kieselgel, Methylenchlorid/Methanol/konzentrierte, wäßrige Ammoniaklösung = 90:10:0.1)
Massenspektrum (ESI⁺): m/z = 581, 583 [M-H]⁺

### II.) 4-[(3-Chlor-4-fluor-phenyl)amino]-7-[3-(2,2-dimethyl-6-oxo-morpholin-4-yl)-propyloxy]-6-[(vinylcarbonyl)amino]-chinazolin

Zu 101 mg Acrylsäure in 5 ml Tetrahydrofuran unter Stickstoffatmosphäre werden 0.47 ml Triethylamin gegeben. Diese Mischung wird in einem Trockeneis/Aceton-Kühlbad auf etwa -50°C abgekühlt und mit 119 mg Acrylsäurechlorid in 3 ml Tetrahydrofuran versetzt, wobei ein farbloser Niederschlag entsteht. Die Suspension wird noch etwa eine Stunde bei dieser Temperatur gerührt. Anschließend werden 240 mg 6-Amino-4-[(3-chlor-4-fluor-phenyl)amino]-7-[3-(2,2-dimethy]-6-oxo-morpholin-4-yl)-propyloxy]-chinazolin in 7 ml Tetrahydrofuran bei -55°C zugetropft. Man läßt das Reaktionsgemisch im Kühlbad langsam auf -30°C erwärmen. Nach etwa einer Stunde wird das Trockeneis/Aceton-Kühlbad gegen ein Eis/Natriumchlorid-Kühlbad ausgetauscht. Man läßt das Reaktionsgemisch darin auf 0°C erwärmen. Sobald die Umsetzung vollständig ist, wird die Reaktion mit Wasser und Methylenchlorid versetzt und mit Natronlauge alkalisch gestellt. Die abgetrennte wäßrige Phase wird nochmals mit Methylenchlorid und wenig Methanol extrahiert. Die vereinigten organischen Extrakte werden mit Wasser gewaschen, getrocknet und eingeengt. Es bleibt ein gelbes Harz zurück, welches über eine Kieselgelsäule mit Methylenchlorid/Methanol (98:2) als Laufmittel chromatographiert wird. Das gewünschte Produkt wird mit wenig *tert*.Butylmethyether verrührt, der feinkristalline Niederschlag wird abgesaugt, mit *tert*.Butylmethyether nachgewaschen und bei 50°C im Vakuum getrocknet.
Ausbeute: 160 mg (60 % der Theorie),
R_{f}-Wert: 0.42 (Kieselgel, Methylenchlorid/Methanol = 95:5)
Massenspektrum (ESI⁺): m/z = 526, 528 [M-H]⁺

Analog zu II.) werden folgende Verbindungen erhalten:
(1) 4-[(3-Chlor-4-fluor-phenyl)amino]-7-[2-((S)-6-methyl-2-oxo-morpholin-4-yl)-ethoxy]-6-[(vinylcarbonyl)amino]-chinazolin
   R_{f}-Wert: 0.32 (Kieselgel, Methylenchlorid/Methanol = 95:5)
   Massenspektrum (ESI⁺): m/z = 498, 500 [M-H]⁺
(2) 4-[(3-Chlor-4-fluor-phenyl)amino]-7-[4-((R)-6-methyl-2-oxo-morpholin-4-yl)-butyloxy]-6-[(vinylcarbonyl)amino]-chinazolin
   R_{f}-Wert: 0.30 (Kieselgel, Methylenchlorid/Methanol = 95:5)
   Massenspektrum (ESI⁺): m/z = 550, 552 [M+Na]⁺
(3) 4-[(3-Chlor-4-fluor-phenyl)amino]-7-[4-((*S*)-6-methyl-2-oxo-morpholin-4-yl)-butyloxy]-6-[(vinylcarbonyl)amino]-chinazolin
   Massenspektrum (ESI⁺): m/z = 526, 528 [M-H]⁺

### III.) 4-[(3-Chlor-4-fluorphenyl)amino]-6-{[4-(N,N-diethylamino)-1-oxo-2-buten-1-yl]amino}-7-cyclopropylmethoxy-chinazolin

Zu einer Lösung aus 640 mg 4-Brom-2-butensäure in 10 ml Methylenchlorid werden bei Raumtemperatur 0.67 ml Oxalylchlorid und ein Tropfen Dimethylformamid gegeben Das Reaktionsgemisch wird noch ca. eine halbe Stunde bei Raumtemperatur gerührt, bis die Gasentwicklung beendet ist. Das entstandene Säurechlorid wird am Rotationsverdampfer im Vakuum weitgehend vom Lösungsmittel befreit. Anschließend wird das Rohprodukt in 10 ml Methylenchlorid gelöst und unter Eisbad-Kühlung zu einer Mischung aus 1.00 g 6-Amino-4-[(3-chlor-4-fluorphenyl)-amino]-7-cyclopropylmethoxy-chinazolin und 1.60 ml Hünigbase in 50 ml Tetrahydrofuran getropft. Das Reaktionsgemisch wird 1.5 Stunden im Eisbad und weitere 2 Stunden bei Raumtemperatur gerührt. Dann werden 2.90 ml Diethylamin zugesetzt und das Gemisch wird 2.5 Tage bei Raumtemperatur gerührt. Zur Aufarbeitung wird das Reaktionsgemisch filtriert und das Filtrat eingeengt. Der Kolbenrückstand wird chromatographisch über eine Kieselgelsäule mit Essigester/Methanol (19:1) gereinigt.
Ausbeute: 550 mg (40 % der Theorie)
Schmelzpunkt: 114°C
Massenspektrum (ESI⁺): m/z = 498, 500 [M+H]⁺

Analog zu III.) werden die folgenden Verbindungen erhalten:
(1) 4-[(3-Chlor-4-fluor-phenyl)amino]-6-{[4-(morpholin-4-yl)-1-oxo-2-buten-1-yl]-amino}-7-cyclopropylmethoxy-chinazolin
   R_{f}-Wert: 0.53 (Kieselgel, Essigester/Methanol =9:1)
   Massenspektrum (ESI⁺): m/z = 510, 512 [M-H]⁺
(2) 4-[(3-Chlor-4-fluorphenyl)amino]-6-{[4-(N,N-dimethylamino)-1-oxo-2-buten-1-yl]-amino}-7-cyclopropylmethoxy-chinazolin
   Schmelzpunkt: 137°C
   Massenspektrum (ESI⁺): m/z = 470, 472 [M+H]⁺
(3) 4-[(*R*)-(1-Phenyl-ethyl)amino]-6-{[4-(morpholin-4-yl)-1-oxo-2-buten-1-yl]amino}-7-cyclopropylmethoxy-chinazolin
   R_{f}-Wert: 0.37 (Kieselgel, Essigester/Methanol =9:1)
   Massenspektrum (ESI⁺): m/z = 488 [M+H] ⁺
(4) 4-[(R)-(1-Phenyl-ethyl)amino]-6-{[4-(morpholin-4-yl)-1-oxo-2-buten-1-yl]amino}-7-cyclopentyloxy-chinazolin
   R_{f}-Wert: 0.35 (Kieselgel, Essigester/Methanol =9:1)
   Massenspektrum (ESI⁺): m/z = 502 [M+H]⁺

### IV.) 4-[(3-Methylphenyl)amino]-6-[(4-(N-[(ethoxycarbonyl)methyl]-N-methylamino}-1-oxo-2-buten-1-yl)amino]-7-methoxy-chinazolin

Zu einer Lösung aus 842 mg 4-Brom-2-butensäure in 15 ml Methylenchlorid werden bei Raumtemperatur 0.86 ml Oxalylchlorid und ein Tropfen Dimethylformamid gegeben. Das Reaktionsgemisch wird noch ca. eine Stunde bei Raumtemperatur gerührt, bis die Gasentwicklung beendet ist. Das entstandene Säurechlorid wird am Rotationsverdampfer im Vakuum weitgehend vom Lösungsmittel befreit. Anschließend wird das Rohprodukt in 10 ml Methylenchlorid aufgenommen und unter Eisbad-Kühlung innerhalb von fünf Minuten zu einer Mischung aus 1.0 g 6-Amino-4-[(3-methylphenyl)amino]-7-methoxy-chinazolin und 2.0 ml Hünigbase in 50 ml Tetrahydrofuran getropft. Das Reaktionsgemisch wird zwei Stunden unter Eisbad-Kühlung und noch zwei weitere Stunden bei Raumtemperatur gerührt. Anschließend werden 6.7 ml Hünigbase, 5.48 g Sarcosinethylesterhydrochlorid und 3 ml Dimethylformamid zugegeben und das Ganze über Nacht bei Raumtemperatur gerührt. Zur Aufarbeitung wird das Reaktionsgemisch am Rotationsverdampfer im Vakuum eingeengt und der Kolbenrückstand zwischen 75 ml Essigester und 75 ml Wasser verteilt. Die organische Phase wird mit Wasser und gesättigter Natriumchlorid-Lösung gewaschen, über Magnesiumsulfat getrocknet und eingeengt. Das Rohprodukt wird chromatographisch über eine Kieselgelsäule mit Methylenchlorid/Methanol (20: 1) gereinigt.
Aubeute: 326 mg (20 % der Theorie)
Schmelzpunkt: 122-124°C
Massenspektrum (ESI⁺): m/z = 464 [M+H]⁺

Analog zu IV.) wird folgende Verbindung erhalten: 4-[(3-Chlor-4-fluorphenyl)amino]-6-[(4-{N-[2-(ethoxycarbanyl)-ethyl]-N-[(ethoxycarbonyl)methyl]amino}-1-oxo-2-buten-1-yl)amino]-7-cyclopropylmethoxy-chinazolin
R_{f}-Wert: 0.62 (Aluminiumoxid, Cyclohexan/Essigester = 1:1)
Massenspektrum (EI): m/z = 627, 629 [M] ⁺

### V.) 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin

950 mg 4-[(3-Chlor-4-fluor-phenyl)amino]-6-[(4-{N-[(ethoxycarbonyl)methyl]-N-((R)-2-hydroxy-3-methoxy-propyl)-amino}-1-oxo-2-buten-1-yl)amino]-7-cyclopropylmethoxy-chinazolin und 195 µl Methansulfonsäure in 10 ml Acetonitril werden etwa vier Stunden unter Rückfluß erhitzt. Zur Aufarbeitung wird das Reaktionsgemisch in einem Eiswasserbad abgekühlt, mit 75 ml Essigester und 25 ml gesättigter Natriumhydrogencarbonat-Lösung versetzt und 10 Minuten kräftig durchgerührt. Die organische Phase wird abgetrennt, mit gesättigter Natriumhydrogencarbonat-Lösung und gesättigter Natriumchlorid-Lösung gewaschen und über Magnesiumsulfat getrocknet. Das Lösungsmittel wird im Vakuum abdestilliert, wobei ein bräunlicher Schaum zurückbleibt.
Ausbeute: 610 mg (69 % der Theorie),
R_{f}-Wert: 0.55 (Kieselgel, Methylenchlorid/Methanol = 9:1)
Massenspektrum (ESI⁺): m/z = 570, 572 [M+H]⁺

### VI.) 4-[(3-Chlor-4-fluor-phenyl)amino]-6-{[4-((S)-6-methyl-2-oxo-morpholin-4-yl)-1-oxo-2-buten-1-yl]amino}-7-cyclopropylmethoxy-chinazolin

Eine Gemisch aus 700 mg 4-[(3-Chlor-4-fluor-phenyl)amino]-6-[(4-{N-[(*tert*.butyloxycarbonyl)methyl]-N-((*S*)-2-hydroxy-prop-1-yl)-amino}-1-oxo-2-buten-1-yl)amino]-7-cyclopropylmethoxy-chinazolin und 228 mg p-Toluolsulfonsäure-hydrat in 20 ml Acetonitril wird fünf Stunden unter Rückfluß erhitzt. Dann werden weitere 200 mg p-Toluolsulfonsäure-hydrat zugegeben und es wird nochmals fünf Stunden unter Rückfluß erhitzt. Zur Aufarbeitung wird das Reaktionsgemisch zur Trockne eingeengt. Der Kolbenrückstand wird zwischen Essigester und gesättigter Natriumcarbonat-Lösung verteilt. Die organische Phase wird abgetrennt, mit gesättigter Natriumcarbonat-Lösung, Wasser und gesättigter Natriumchlorid-Lösung gewaschen, über Magnesiumsulfat getrocknet und eingeengt. Der ölige Rückstand wird durch Verrühren mit 15 ml Diethylether zur Kristallisation gebracht.
Schmelzpunkt: 173-175°C
Massenspektrum (ESI⁺): m/z = 540, 542 [M+H]⁺

Analog zu Vl.) werden die folgenden Verbindungen erhalten:
(1) 4-[(3-Chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazolin
   R_{f}-Wert: 0.54 (Kieselgel, Methylenchlorid/Methanol = 9:1)
   Massenspektrum (ESI⁺): m/z = 540, 542 [M+H]⁺
(2) 4-[(3-Chlor-4-fluor-phenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazolin
   (Die Reaktion wird mit Methansulfonsäure in Acetonitril durchgeführt)
   R_{f}-Wert: 0.38 (Kieselgel, Methylenchlorid/Methanol = 9:1)
   Massenspektrum (ESI⁺): m/z = 556, 558 [M+H]⁺

### VII.) 4-[(3-Brom-phenyl)amino]-6-[2-((S)-6-methyl-2-oxo-morpholin-4-yl)-ethoxy]-7-methoxy-chinazolin

Zu 380 mg 4-[(3-Brom-phenyl)amino]-6-(2-{N-[(*tert*.butyloxycarbonyl)methyl]-N-((*S*)-2-hydroxy-propyl)-amino}-ethoxy)-7-methoxy-chinazolin in 8 ml Acetonitril werden 90 *µ*l Methansulfonsäure gegeben. Das Reaktionsgemisch wird ca. drei Stunden unter Rückfluß erhitzt, dann wird nochmals ein Äquivalent Methansulfonsäure zugegeben und weiter unter Rückfluß erhitzt, bis die Umsetzung vollständig ist. Zur Aufarbeitung wird das Reaktionsgemisch mit Essigester verdünnt und mit gesättigter Natriumhydrogencarbonat-Lösung und gesättigter Natriumchlorid-Lösung gewaschen. Die organische Phase wird über Magnesiumsulfat getrocknet und im Vakuum eingeengt. Der Kolbenrückstand wird mit Diethylether verrührt und abgesaugt. Man erhält die Titelverbindung als weißen Feststoff.
Ausbeute: 280 mg (85 % der Theorie),
Schmelzpunkt: 190°C
Massenspektrum (ESI⁺): m/z = 485, 487 [M-H]⁺

Analog zu Vll.) wird folgende Verbindung erhalten:
4-[(3-Chlor-4-fluor-phenyl)amino]-6-[2-((S)-6-methyl-2-oxo-morpholin-4-yl)-ethoxy]-7-methoxy-chinazolin
   (Die Reaktion wird mit Trifluoressigsäure in Acetonitril durchgeführt)
   Schmelzpunkt: 212-213°C
   Massenspektrum (ESI⁺): m/z = 461, 463 [M+H]⁺

### VIII.) 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin

Zu einer Lösung aus 4.50 g Bromcrotonsäure in 60 ml Methylenchlorid werden 4.70 ml Oxalylchlorid getropft. Anschließend wird ein Tropfen N,N-Dimethylformamid zugegeben. Nach ca. 30 Minuten ist die Gasentwicklung beendet und das Reaktionsgemisch wird am Rotationsverdampfer eingeengt. Das rohe Bromcrotonsäurechlorid wird in 30 ml Methylenchlorid aufgenommen und unter Eisbad-Kühlung zu einer Lösung aus 7.00 g 4-[(3-Chlor-4-fluorphenyl)amino]-6-amino-7-cyclopropylmethoxy-chinazolin und 10.20 ml Hünigbase in 150 ml Tetrahydrofuran getropft. Das Reaktionsgemisch wird etwa 1.5 Stunden unter Eisbadkühlung und weitere zwei Stunden bei Raumtemperatur gerührt. Nun werden 5.20 g N-(2-Methoxy-ethyl)-N-methyl-amin zugegeben und das Reaktionsgemisch wird über Nacht bei Raumtemperatur gerührt. Zur Aufarbeitung es wird mit Methylenchlorid verdünnt und gründlich mit Wasser gewaschen. Die organische Phase wird über Magnesiumsulfat getrocknet und eingeengt. Das Rohprodukt wird chromatographisch über eine Kieselgelsäule mit Essigester gefolgt von Essigester/Methanol (19:1) als Laufmittel gereinigt.
Ausbeute: 5.07 g (51 % der Theorie)
Massenspektrum (ESI⁺): m/z = 512, 514 [M-H]⁺
R_{f}-Wert: 0.25 (Kieselgel, Essigester/Methanol = 9:1)

Analog zu VIII). werden folgende Verbindungen erhalten:
(1) 4-[(3-Chlor-4-fluorphenyl)amino]-6-{[4-(N,N-dimethylamino)-1-oxo-2-buten-1-yl]-amino}-7-cyclopentyloxy-chinazolin
   Massenspektrum (ESI⁺): m/z = 482, 484 [M-H]⁺
   R_{f}-Wert: 0.11 (Kieselgel, Essigester/Methanol = 9:1)
(2) 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
   Massenspektrum (ESI⁺): m/z = 532 [M-H]⁺
   R_{f}-Wert: 0.40 (Kieselgel, Essigester/Methanol =9:1)
(3) 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
   Massenspektrum (ESI⁺): m/z = 502 [M-H]⁺
   R_{f}-Wert: 0.20 (Kieselgel, Essigester/Methanol =9:1)
(4) 4-[(R)-(1 -Phenyl-ethyl)amino]-6-({4-[N-(2-methoxy-ethyl)-N-methyl-amino]-1-oxo-2-buten-1-yl}amino)-7-cyclopropylmethoxy-chinazolin
   Massenspektrum (ESI⁺): m/z = 488 [M-H]⁺
   R_{f}-Wert: 0.25 (Kieselgel, Essigester/Methanol =9:1)
(5) 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
   Massenspektrum (ESI⁺): m/z = 514 [M-H]⁺
   R_{f}-Wert: 0.15 (Kieselgel, Essigester/Methanol = 9:1)
(6) 4-[(3-Chlor-4-fluorphenyl)amino]-6-{[4-(N,N-dimethylamino)-1-oxo-2-buten-1-yl]-amino}-7-((R)-tetrahydrofuran-3-yloxy)-chinazolin
   Massenspektrum (ESI⁺): m/z = 486, 488 [M+H]⁺
(7) 4-[(3-Chlor-4-fluorphenyl)amino]-6-{[4-(N,N-dimethylamino)-1-oxo-2-buten-1-yl]-amino}-7-((*S*)-tetrahydrofuran-3-yloxy)-chinazolin
   Massenspektrum (ESI⁺). m/z = 486, 488 [M+H]⁺
   R_{f}-Wert: 0.45 (Kieselgel, Methylenchlorid/Methanol = 5:1)
(8) 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazoün
   Massenspektrum (ESI⁺): m/z = 528, 530 [M-H]⁺
   R_{f}-Wert: 0.25 (Kieselgel, Essigester/Methanol = 9:1)
(9) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
   Massenspektrum (ESI⁺): m/z = 508, 510 [M-H]⁺
   Schmelzpunkt: 140°C
(10) 4-[(3-Chlor-4-fluorphenyl)amino]-6-{[4-(N,N-dimethylamino)-1-oxo-2-buten-1-yl]-amino}-7-[(*R*)-(tetrahydrofuran-2-yl)methoxy]-chinazolin
   Massenspektrum (ESI⁺): m/z = 500, 502 [M+H]⁺
   Schmelzpunkt: 110-112°C
(11) 4-[(3-Chlor-4-fluorphenyl)amino]-6-{[4-(N,N-dimethylamino)-1-oxo-2-buten-1-yl]-amino}-7-[(*S*)-(tetrahydrofuran-2-yl)methoxy]-chinazolin
   Massenspektrum (ESI⁺): m/z = 500, 502 [M+H]⁺
   R_{f}-Wert: 0.23 (Kieselgel, Essigester/Methanol/konz. wässriges Ammoniak = 90:10:0.1)

Nachfolgend werden erfindungsgemäß besonders bevorzugte Formulierungen, enthaltend die beiden Komponenten 1 und 2 beschrieben, ohne allerdings den Kern der Erfindung auf selbige zu beschränken.

### Formulierungsbeispiele

### Inhalationspulver:

1)

| **Bestandteile** | ***µ*g pro Kapsel** |
|---|---|
| **1**'-Bromid | 60 |
| EG FR-Kinase-Hemmer **2** | 3500 |
| Lactose | 3440 |
| Summe | 7000 |

2)

| **Bestandteile** | ***µ*g pro Kapsel** |
|---|---|
| **1**'-Bromid | 100 |
| EGFR-Kinase-Hemmer **2** | 3000 |
| Lactose | 3900 |
| Summe | 7000 |

3)

| **Bestandteile** | ***µ*g pro Kapsel** |
|---|---|
| **1**'-Bromid | 150 |
| EGFR-Kinase-Hemmer **2** | 5000 |
| Lactose | 4850 |
| Summe | 10000 |

## Patentansprüche

1. Arzneimittel **gekennzeichnet durch** einen Gehalt an einem oder mehreren Anticholinergika der Formel **1** 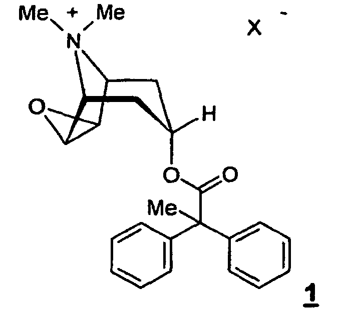
worin
X - ein einfach negativ geladenes Anion, vorzugsweise ein Anion ausgewählt aus der Gruppe bestehend aus Chlorid, Bromid, lodid, Sulfat, Phosphat, Methansulfonat, Nitrat, Maleat, Acetat, Citrat, Fumarat, Tartrat, Oxalat, Succinat, Benzoat und p-Toluolsulfonat bedeutet,
in Kombination mit einem oder mehreren EGFR-Kinase-Hemmer **(2),** gegebenenfalls in Form ihrer Enantiomere, Gemische der Enantiomere oder in Form der Racemate, gegebenenfalls in Form der Solvate oder Hydrate sowie gegebenenfalls gemeinsam mit einem pharmazeutisch verträglichen Hilfsstoff.

2. Arzneimittel nach Anspruch 1, **dadurch gekennzeichnet, daß** die Wirkstoffe **1** und **2** entweder gemeinsam in einer einzigen Darreichungsform oder in zwei getrennten Darreichungsformen enthalten sind.

3. Arzneimittel nach einem der Ansprüche 1 oder 2, **dadurch gekennzeichnet, daß** in den Verbindungen der Formel **1** X⁻ ein einfach negativ geladenes Anion ausgewählt aus der Gruppe Chlorid, Bromid, p-Toluolsulfonat und Methansulfonat bedeutet.

4. Arzneimittel nach einem der Ansprüche 1 bis 3, **dadurch gekennzeichnet, daß** in den Verbindungen der Formel **1** X⁻ für Bromid steht.

5. Arzneimittel nach einem der Ansprüche 1 bis 4, **dadurch gekennzeichnet, daß** **2** ausgewählt ist aus der Gruppe bestehend aus
4-[(3-Chlor-4-fluor-phenyl)amino]-7-(2-{4-[(S)-(2-oxo-tetrahydrofuran-5-yl)-carbonyl]-piperazin-1-yl}-ethoxy)-6-[(vinylcarbonyl)amino]-chinazolin,
4-[(3-Chlor-4-fluor-phenyl)amino]-7-[2-((*S*)-6-methyl-2-oxo-morpholin-4-yl)-ethoxy]-6-[(vinylcarbonyl)amino]-chinazolin,
4-[(3-Chlor-4-fluor-phenyl)amino]-7-[4-((*R*)-6-methyl-2-oxo-morpholin-4-yl)-butyloxy]-6-[(vinylcarbonyl)amino]-chinazolin,
4-[(3-Chlor-4-fluor-phenyl)amino]-7-[4-((*S*)-6-methyl-2-oxo-morpholin-4-yl)-butyloxy]-6-[(vinylcarbonyl)amino]-chinazolin,
4-[(3-Chlor-4-fluorphenyl)amino]-6-{[4-(morpholin-4-yl)-1-oxo-2-buten-1-yl]-amino}-7-cyclopropylmethoxy-chinazolin,
4-[(3-Chlor-4-fluorphenyl)amino]-6-{[4-(N,N-diethylamino)-1-oxo-2-buten-1-yl]amino}-7-cyclopropylmethoxy-chinazolin,
4-[(3-Chlor-4-fluorphenyl)amino]-6-{[4-(N,N-dimethylamino)-1-oxo-2-buten-1-yl]amino}-7-cyclopropylmethoxy-chinazolin,
4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
4-[(*R*)-(1-Phenyl-ethyl)amino]-6-{[4-(morpholin-4-yl)-1-oxo-2-buten-1-yl]-amino}-7-cyclopropylmethoxy-chinazolin,
4-[(*R*)-(1-Phenyl-ethyl)amino]-6-{[4-(morpholin-4-yl)-1-oxo-2-buten-1-yl]-amino}-7-cyclopentyloxy-chinazolin,
4-[(3-Chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazolin,
4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin,
4-[(3-Chlor-4-fluor-phenyl)amino]-6-{[4-{(*R*)-2-methoxymethyl-6-oxo-morpholin-4-yl)-1-oxo-2-buten-1-yl]amino}-7-cyclopropylmethoxy-chinazolin,
4-[(3-Chlor-4-fluor-phenyl)amino]-6-[2-((*S*)-6-methyl-2-oxo-morpholin-4-yl)-ethoxy]-7-methoxy-chinazolin,
4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
4-[(3-Chlor-4-fluorphenyl)amino]-6-{[4-(N,N-dimethylamino)-1-oxo-2-buten-1-yl]amino}-7-cyclopentyloxy-chinazolin,
4-[(*R*)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
4-[(*R*)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
4-[(3-Chlor-4-fluorphenyl)amino]-6-{[4-(N, N-dimethylamino)-1-oxo-2-buten-1-yl]amino}-7-((*R*)-tetrahydrofuran-3-yloxy)-chinazolin,
4-[(3-Chlor-4-fluorphenyl)amino]-6-{[4-(N, N-dimethylamino)-1-oxo-2-buten-1-yl]amino}-7-((*S*)-tetrahydrofuran-3-yloxy)-chinazolin,
4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
4-[(3-Chlor-4-fluorphenyl)amino]-6-{[4-(N-cyclopropyl-N-methyl-amino)-1-oxo-2-buten-1-yl]amino}-7-cyctopentyloxy-chinazolin,
4-[(3-Chlor-4-fluorphenyl)aminoJ-6-{[4-(N,N-dimethylamino)-1-oxo-2-buten-1-yl]amino}-7-[(R)-(tetrahydrofuran-2-yl)methoxy]-chinazolin,
4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
4-[(3-Chlor-4-fluorphenyl)amino]-6-[3-(morpholin-4-yl)-propyloxy]-7-methoxy-chinazolin,
4-[(3-Ethinyl-phenyl)amino]-6,7-bis-(2-methoxy-ethoxy)-chinazolin,
4-[(3-Chlor-4-fluorphenyl)amino]-7-[3-(morpholin-4-yl)-propyloxy]-6-[(vinyl-carbonyl)amino]-chinazolin,
4-[(*R*)-(1-Phenyl-ethyl)amino]-6-(4-hydroxy-phenyl)-7H-pyrrolo[2,3-d]pyrimidin,
3-Cyano-4-[(3-chlor-4-fluorphenyl)amino]-6-{[4-(N,N-dimethylamino)-1-oxo-2-buten-1-yl]amino}-7-ethoxy-chinolin,
4-{[3-Chlor-4-(3-fluor-benzyloxy)-phenyl]amino}-6-(5-{[(2-methansulfonyl-ethyl)amino]methyl}-furan-2-yl)chinazolin, Cetuximab, Trastuzumab, ABX-EGF und Mab ICR-62, gegebenenfalls in Form ihrer physiologisch verträglichen Säureadditionssalze.

6. Arzneimittel nach einem der Ansprüche 1 bis 5, **dadurch gekennzeichnet, daß** 2 ausgewählt ist aus der Gruppe bestehend aus
4-[(3-Chlor-4-fluor-phenyl)amino]-7-(2-{4-[(S)-(2-oxo-tetrahydrofuran-5-yl)-carbonyl]-piperazin-1-yl}-ethoxy)-6-[(vinylcarbonyl)amino]-chinazolin,
4-[(3-Chlor-4-fluor-phenyl)amino]-7-[2-((S)-6-methyl-2-oxo-morpholin-4-yl)-ethoxy]-6-[(vinytcarbonyl)amino]-chinazolin,
4-[(3-Chlor-4-fluor-phenyl)amino]-7-[4-((R)-6-methyl-2-oxo-morpholin-4-yl)-butyloxy]-6-[(vinylcarbonyl)amino]-chinazolin,
4-[(3-Chlor-4-fluor-phenyl)amino]-7-[4-((S)-6-methyl-2-oxo-morpholin-4-yl)-butyloxy]-6-[(vinylcarbonyl)amino]-chinazolin,
4-[(3-Chlor-4-fluor-phenyl)amino]-7-[4-(2,2-dimethyl-6-oxo-morpholin-4-yl)-butyloxy]-6-[(vinylcarbonyl)amino]-chinazolin,
4-[(3-Chlor-4-fluorphenyl)amino]-6-{[4-(morpholin-4-yl)-1-oxo-2-buten-1-yl]-amino}-7-cyclopropylmethoxy-chinazolin,
4-[(3-Chlor-4-fluorphenyl)amino]-6-{[4-(N,N-diethylamino)-1 -oxo-2-buten-1 - yl]amino}-7-cyclopropylmethoxy-chinazolin,
4-[(3-Chlor-4-fluorphenyf)amino]-6-{[4-(N,N-dimethylamino)-1-oxo-2-buten-1-yl]amino}-7-cyclopropylmethoxy-chinazolin,
4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
4-[(R)-(1-Phenyl-ethyl)amino]-6-{[4-(morpholin-4-yl)-1-oxo-2-buten-1-yl]-amino}-7-cyclopropylmethoxy-chinazolin,
4-[(R)-(1-Phenyl-ethyl)amino]-6-{[4-(morpholin-4-yl)-1-oxo-2-buten-1-yl]-amino}-7-cyclopentyloxy-chinazolin,
4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin,
4-[(3-Chlor-4-fluor-phenyl)amino]-6-({4-[bis-(2-methoxyethyl)-amino]-1-oxo-2-buten-1-yl}amino)-7-cyclopropylmethoxy-chinazolin,
4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin,
4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin,
4-[(3-Chlor-4-fluor phenyl)amino]-6-[2-((S)-6-methyl-2-oxo-morpholin-4-yl)-ethoxy]-7-methoxy-chinazolin,
4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
4-[(3-Chlor-4-fluorphenyl)amino]-6-{[4-(N,N-dimethylamino)-1-oxo-2-buten-1-yl]amino}-7-cyclopentyloxy-chinazolin.
4-[(3-Chlor-4-fluor-phenyl)amino]-6-{[4-((S)-2-methoxymethyl-6-oxo-morpholin-4-yl)-1-oxo-2-buten-1-yl]amino}-7-cyclopropylmethoxy-chinazolin,
4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
4-[(R)-(1-Phenyl-ethyl)amino]-6-({4-[N-(2-methoxy-ethyl)-N-methyl-amino]-1-oxo-2-buten-1-yl)amino)-7-cyclopropylmethoxy-chinazolin,
4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
4-[(3-Chlor-4-fluorphenyl)amino]-6-{[4-(N,N-dimethylamino)-1-oxo-2-buten-1-yl]amino}-7-((R)-tetrahydrofuran-3-ytoxy)-chinazolin,
4-[(3-Chlor-4-fluorphenyl)amino]-6-{[4-(N,N-dimethytamino)-1-oxo-2-buten-1-yl]amino}-7-((S)-tetrahydrofuran-3-yloxy)-chinazolin,
4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin,
4-[(3-Chlor-4-fluorphenyl)amino]-6-{[4-{N,N-dimethylamino)-1-oxo-2-buten-1-yl]amino}-7-[(S)-(tetrahydrofuran-2-yl)methoxy]-chinazolin,
4-[(3-Chlor-4-fluor-phenyl)amino]-6-[(4-dimethylamino-cyclohexyl)amino]-pyrimido[5,4-d]pyrimidin und
4-[(3-Chlor-4-fluorphenyl)amino]-6-[3-(morpholin-4-yl)-propyloxy]-7-methoxy-chinazolin, gegebenenfalls in Form ihrer physiologisch verträglichen Säureadditionssalze.

7. Arzneimittel nach einem der Ansprüche 1 bis 6, **dadurch gekennzeichnet, daß 2** ausgewählt ist aus der Gruppe bestehend aus
4-[(3-Chlor-4-fluor-phenyl)amino]-7-[4-((R)-6-methyl-2-oxo-morpholin-4-yl}-butyloxy]-6-[(vinylcarbonyl)amino]-chinazolin,
4-[(3-Chlor-4-fluor-phenyl)amino]-7-[4-((S)-6-methyl-2-oxo-morpholin-4-yl)-butyloxy]-6-[(vinylcarbonyl)aminol-chinazolin,
4-[(3-Chlor-4-fluor-phenyl)amino]-7-(2-{4-[(S)-(2-oxo-tetrahydrofuran-5-yl)-carbonyl]-piperazin-1-yl}-ethoxy)-6-[(vinylcarbonyl)amino]-chinazolin,
4-[(3-Chlor-4-fluor-phenyl)amino]-7-[2-((S)-6-methyl-2-oxo-morpholin-4-yl)-ethoxy]-6-[(vinylcarbonyl)amino]-chinazolin,
4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
4-[(R)-(1-Phenyl-ethyl)amino]-6-{[4-(morpholin-4-yl)-1-oxo-2-buten-1-yl]-amino}-7-cyclopropylmethoxy-chinazolin und
4-[(3-Chlor-4-fluorphenyl)amino]-6-[3-(morpholin-4-yl)-propyloxy]-7-methoxy-chinazolin, gegebenenfalls in Form ihrer physiologisch verträglichen Säureadditionssalze.

8. Arzneimittel nach einem der Ansprüche 1 bis 7, **dadurch gekennzeichnet, daß** es in Form einer für die Inhalation geeigneten Darreichungsform vorliegt.

9. Arzneimittel nach Anspruch 8, **dadurch gekennzeichnet, daß** es sich um eine Darreichungsform ausgewählt aus der Gruppe Inhalationspulver, treibgashaltige Dosieraerosole und treibgasfreie Inhalationslösungen oder -suspensionen handelt.

10. Arzneimittel nach Anspruch 9, **dadurch gekennzeichnet, daß** es ein Inhalationspulver ist, welches **1** und **2** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hiffsstoffe miteinandenthält.

11. Arzneimittel nach Anspruch 9, **dadurch gekennzeichnet, daß** es ein Inhalationspulver ist, welches als Bestandteile lediglich die Wirkstoffe **1** und **2** enthält.

12. Arzneimittel nach Anspruch 9, **dadurch gekennzeichnet, daß** es sich um ein treibgashaltiges Inhalationsaerosol handelt, welches **1** und **2** in gelöster oder dispergierter Form enthält.

13. Arzneimittel nach Anspruch 12, **dadurch gekennzeichnet, daß** es als Treibgas Kohlenwasserstoffe wie n-Propan, n-Butan oder Isobutan oder Halogenkohlenwasserstoffe wie chlorierte und/oder fluorierte Derivate des Methans, Ethans, Propans, Butans, Cyclopropans oder Cyclobutans enthält.

14. Arzneimittel nach Anspruch 13, **dadurch gekennzeichnet, daß** das Treibgas TG11, TG12, TG134a, TG227 oder Gemische davon, bevorzugt TG134a, TG227 oder ein Gemisch davon darstellt.

15. Arzneimittel nach Anspruch 9, **dadurch gekennzeichnet, daß** es sich um eine treibgasfreie Inhalationslösung oder -suspension handelt, die als Lösemittel Wasser, Ethanol oder ein Gemisch aus Wasser und Ethanol enthält.

16. Arzneimittel nach Anspruch 15, **dadurch gekennzeichnet, daß** der pH 2 - 7, bevorzugt 2-5 beträgt.

17. Verwendung einer Zusammensetzung nach einem der Ansprüche 1 bis 16 zur Herstellung eines Medikaments zur Behandlung von entzündlichen und/oder obstruktiven Atemwegserkrankungen.

## Claims

1. Pharmaceutical composition, **characterised in that** it contains one or more anticholinergics of formula **1** 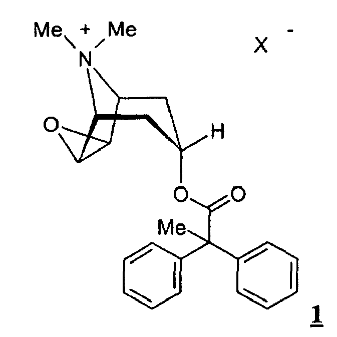 wherein
X⁻ denotes an anion with a single negative charge, preferably an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate,
combined with one or more EGFR kinase inhibitors (**2**), optionally in the form of the enantiomers, mixtures of the enantiomers or in the form of the racemates thereof, optionally in the form of the solvates or hydrates and optionally together with a pharmaceutically acceptable excipient.

2. Pharmaceutical composition according to claim 1, **characterised in that** the active substances **1** and **2** are present either together in a single formulation or in two separate formulations.

3. Pharmaceutical composition according to one of claims 1 and 2, **characterised in that** in the compounds of formula **1** X- is a negatively charged anion selected from the group consisting of chloride, bromide, p-toluenesulphonate and methanesulphonate.

4. Pharmaceutical composition according to one of claims 1 to 3, **characterised in that** in the compounds of formula 1 X denotes bromide.

5. Pharmaceutical composition according to one of claims 1 to 4, **characterised in that** **2** is selected from among :
4-[(3-chloro-4-fluoro-phenyl)amino]-7-(2-{4-[(S)-(2-oxo-tetrahydrofuran-5-yl)carbonyl]-piperazin-1-yl}-ethoxy)-6-[(vinylcarbonyl)amino]-quinazoline,
4-[(3-chloro-4-fluoro-phenyl)amino]-7-[2-((*S*)-6-methyl-2-oxo-morpholin-4-yl)-ethoxy]-6-[(vinylcarbonyl)amino]-quinazoline,
4-[(3-chloro-4-fluoro-phenyl)amino]-7-[4-((*R*)-6-methyl-2-oxo-morpholin-4-yl)-butyloxy]-6-[(vinylcarbonyl)amino]-quinazoline,
4-[(3-chloro-4-fluoro-phenyl)amino]-7-[4-((*S*)-6-methyl-2-oxo-morpholin-4-yl)-butyloxy]-6-[(vinylcarbonyl)amino]-quinazoline,
4-[(3-chloro-4-fluorophenyl)amino]-6-{[4-(morpholin-4-yl)-1-oxo-2-buten-1-yl]amino}-7-cyclopropylmethoxy-quinazoline,
4-[(3-chloro-4-fluorophenyl)amino]-6-{[4-(N,N-diethylamino)-1-oxo-2-buten-1-yl)amino}-7-cyclopropylmethoxy-quinazoline,
4-[(3-chloro-4-fluorophenyl)amino]-6-{[4-(N,N-dimethylamino)-1-oxo-2-buten-1-yl]amino}-7-cyaopropylmethoxy-quinazoline,
4-[(3-chloro-4-fluorophenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-quinazoline,
4-[(*R*)-(1-phenyl-ethyl)amino]-6-{[4-(morpholin-4-yl)-1-oxo-2-buten-1-yl]amino}-7-cyclopropylmethoxy-quinazoline,
4-[(*R*)-(1-phenyl-ethyl)amino]-6-{[4-(morpholin-4-yl)-1-oxo-2-buten-1-yl]amino}-7-cyclopentyloxy-quinazoline,
4-[(3-chloro-4-fluoro-phenyl)amino]-6-{(4-((*R*)-6-methyl-2-oxo-morpholin-4-yl)-1-oxo-2-buten-1-yl]amino}-7-cyclopropylmethoxy-quinazoline,
4-[(3-chloro-4-fluoro-phenyl)amino]-6-{[4-((R)-6-methyl-2-oxo-morpholin-4-yl)-1-oxo-2-buten-1-ylJamino}-7-[(S)-(tetrahydrofuran-3-yl)oxy]-quinazoline,
4-[(3-chloro-4-fluoro-phenyl)amino]-6-{(4-((*R*)-2-methoxymethyl-6-oxo-morpholin-4-yl)-1-oxo-2-buten-1-yl]amino}-7-cyclopropylmethoxy-quinazoline,
4-[(3-chloro-4-fluoro-phenyl)amino]-6-[2-((S)-6-methyl-2-oxo-morpholin-4-yl)-ethoxy]-7-methoxy-quinazoline,
4-[(3-chloro-4-fluorophenyl)amino]-6-({4-[N-(2-methoxy-ethyl)-N-methyl-amino)-1-oxo-2-buten-1-yl} amino)-7-cyclopropylmethoxy-quinazoline,
4-[(3-chloro-4-fluorophenyl)amino]-6-{[4-(N,N-dimethylamino)-1-oxo-2-buten-2-yl]amino}-7-cyclopentyloxy-quinazoline,
4-[(*R*)-(1-phenyl-ethyl)amino]-6-{(4-(N,N-bis-(2-methoxy-ethyl)-amino)-1-oxo-2-buten-1-yl)amino}-7-cyclopropylmethoxy-quinazoline,
4-[(*R*)-(1-phenyl-ethyl)amino]-6-({4-[N-(2-methoxy-ethyl)-N-ethyl-amino]-1-oxo-2-buten-1-yl}amino)-7-cyclopropylmethoxy-quinazoline,
4-((*R*)-(1-phenyl-ethyl)amino)-6-({4-(N-(2-methoxy-ethyl)-N-methyl-amino]-1-oxo-2-buten-1-yl}amino)-7-cyclopropylmethoxy-quinazoline,
4-[(*R*)-(1-phenyl-ethyl)amino]-6-({4-[N-(tetrahydropyran-4-yl)-N-methyl-amino]-1-oxo-2-buten-1-yl} amino)-7-cyclopropylmethoxy-quinazoline,
4-[(3-chloro-4-fluorophenyl)ammo]-6-{[4-(N,N-dimethylamino)-1-oxo-2-buten-1-yl]amino}-7-((R)-tetrahydrofuran-3-yloxy)-quinazoline,
4-[(3-chloro-4-fluorophenyl)amino]-6-{(4-(N,N-dimethylamino)-1-oxo-2-buten-1-yl]amino}-7-((S)-tetrahydrofuran-3-yloxy)-quinazoline,
4-[(3-chloro-4-fluorophenyl)amino]-6-({4-[N-(2-methoxy-ethyl)-N-methyl-amino]-1-oxo-2-buten-1-yl}amino)-7-cyclopentyloxy-quinazoline,
4-[(3-chloro-4-fluorophenyl)amino]-6-{[4-(N-cyclopropyl-N-methyl-amino)-1-oxo-2-buten-1-yl]amino}-7-cyclopentyloxy-quinazoline,
4-[(3-chloro-4-fluorophenyl)amino]-6-{[4-(N,N-dimethylamino)-1-oxo-2-buten-1-yl]amino}-7-[(*R*)-(tetrahydrofuran-2-yl)methoxy]-quinazoline,
4-[(3-chloro-4-fluorophenyl)amino]-6-{[4-(N,N-dimethylamino)-1-oxo-2-buten-1-yl]amino}-7-[(S)-(tetrahydrofuran-2-yl)methoxy)-quinazoline,
4-[(3-chloro-4-fluorophenyl)amino]-6-[3-(morpholin-4-yl)-propyloxy]-7-methoxy-quinazoline,
4-[(3-ethynyl-phenyl)amino]-6,7-bis-(2-methoxy-ethoxy)-quinazoline,
4-[(3-chloro-4-fluorophenyl)amino]-7-[3-(morpholin-4-yl)-propyloxy]-6-[(vinylcarbonyl)amino]-quinazoline,
4-[(*R*)-(1-phenyl-ethyl)amino]-6-(4-hydroxy-phenyl)-7H-pyrrolo[2,3-denotes]pyrimidine,
3-cyano-4-[(3-chloro-4-fluorophenyl)amino]-6-{ [4-(N,N-dimethylamino)-1-oxo-2-buten-1-yl]amino}-7-ethoxy-quinoline,
4-{ [3-chloro-4-(3-fluoro-benzyloxy)-phenyl]amino}-6-(5-{[(2-methanesulphonyl-ethyl)amino]methyl}-furan-2-yl)quinazoline, Cetuximab, Trastuzumab, ABX-EGF and Mab ICR-62, optionally in the form of the physiologically acceptable acid addition salts thereof.

6. Pharmaceutical composition according to one of claims 1 to 5, **characterised in that** **2** is selected from the group consisting of 4-[(3-chloro-4-fluoro-phenyl)amino]-7-(2-{4-[(S)-(2-oxo-tetrahydrofuran-5-yl)carbonyl]-piperazin-1-yl}-ethoxy)-6-[(vinylcarbonyl)amino]-quinazoline,
4-[(3-chloro-4-fluoro-phenyl)amino]-7-[2-((S)-6-methyl-2-oxo-morpholin-4-yl)-ethoxy]-6-[(vinylcarbonyl)amino]-quinazoline,
4-[(3-chloro-4-fluoro-phenyl)amino]-7-[4-((R)-6-methyl-2-oxo-morpholin-4-yl)-butyloxy]-6-[(vinylcarbonyl)amino]-quinazoline,
4-[(3-chloro-4-fluoro-phenyl)amino]-7-[4-((S)-6-methyl-2-oxo-morpholin-4-yl)-butyloxyl-6-[(vinylcarbonyl)amino]-quinazoline,
4-[(3-chloro-4-fluoro-phenyl)amino]-7-[4-(2,2-dimethyl-6-oxo-morpholin-4-yl)-butyloxy]-6-[(vinylcarbonyl)amino]-quinazoline,
4-[(3-chloro-4-fluorophenyl)amino]-6-{[4-(morpholin-4-yl)-1-oxo-2-buten-1-yl]amino}-7-cyclopropylmethoxy-quinazoline,
4-[(3-chloro-4-fluorophenyl)amino]-6-{[4-(N,N-diethylamino)-1-oxo-2-buten-1-yl]amino}-7-cyclopropylmethoxy-quinazoline,
4-[(3-chloro-4-fluorophenyl)amino]-6-{(4-(N,N-dimethylamino)-2-oxo-2-buten-1-yl]amino}-7-cyclopropylmethoxy-quinazoline,
4-[(3-chloro-4-fluorophenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-quinazoline,
4-[(R)-(1-phenyl-ethyl)amino]-6-{[4-(morpholin-4-yl)-1-oxo-2-buten-1-yl]amino}-7-cyclopropylmethoxy-quinazoline,
4-[(R)-(1-phenyl-ethyl)amino]-6-{[4-(morpholin-4-yl)-1-oxo-2-buten-1-yl]amino}-7-cyclopentyloxy-quinazoline,
4-[(3-chloro-4-fluoro-phenyl)amino]-6-{[4-((R)-6-methyl-2-oxo-morpholin-4-yl)-1-oxo-2-buten-1-yl]amino}-7-cyclopropylmethoxy-quinazoline,
4-[(3-chloro-4-fluoro-phenyl)amino]-6-({4-[bis-(2-methoxyethyl)-amino]-1-oxo-2-buten-1-yl}amino)-7-cyclopropylmethoxy-quinazoline,
4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-quinazoline,
4-[(3-chloro-4-fluoro-phenyl)amino]-6-{[4-((R)-2-methoxymethyl-6-oxo-morpholin-4-yl)-1-oxo-2-buten-1-yl]amino}-7-cyclopropylmethoxy-quinazoline,
4-[(3-chloro-4-fluoro-phenyl)amino]-6-[2-((S)-6,methyl-2-oxo-morpholin-4-yl)-ethoxy]-7-methoxy-quinazoline,
4-[(3-chloro-4-fluorophenyl)amino]-6-({4-[N-(2-methoxy-ethyl)-N-methyl-amino]-1-oxo-2-buten-1-yl}amino)-7-cyclopropylmethoxy-quinazoline,
4-[(3-chloro-4-fluorophenyl)amino]-6-{[4-(N,N-dimethylamino)-1-oxo-2-buten-1-yl]amino}-7-cyclopentyloxy-quinazoline,
4-[(3-chloro-4-fluoro-phenyl)amino]-6-{[4-((S)-2-methoxymethyl-6-oxo-morpholin-4-yl)-1-oxo-2-buten-1-yl]amino}-7-cyclopropylmethoxy-quinazoline,
4-[(R)-(1-phenyl-ethyl)amino)-6-{[4-(N,N-bis-(2-methoxy-ethyl)-amino)-1-oxo-2-buten-1-yl]amino}-7-cyclopropylmethoxy-quinazoline,
4-[(R)-(1-phenyl-ethyl)amino]-6-({4-[N-(2-methoxy-ethyl)-N-ethyl-amino]-1-oxo-2-buten-1-yl}amino)-7-cyclopropylmethoxy-quinazoline,
4-[(R)-(1-phenyl-ethyl)amino]-6-({4-[N-(2-methoxy-ethyl)-N-methyl-amino]-1-oxo-2-buten-1-yl}amino)-7-cyclopropylmethoxy-quinazoline,
4-[(R)-(1-phenyl-ethyl)amino]-6-({4-[N-(tetrahydropyran-4-yl)-N-methyl-amino]-1-oxo-2-buten-1-yl} amino)-7-cyclopropylmethoxy-quinazoline,
4-[(3-chloro-4-fluorophenyl)amino]-6-{[4-(N,N-dimethylamino)-1-oxo-2-buten-1-yl]amino}-7-((R)-tetrahydrofuran-3-yloxy)-quinazoline,
4-[(3-chloro-4-fluorophenyl)amino]-6-{[4-(N,N-dimethylamino)-1-oxo-2-buten-2-yl]amino}-7-((S)-tetrahydrofuran-3-yloxy)-quinazoline,
4-[(3-chloro-4-fluorophenyl)amino]-6-({4-[N-(2-methoxy-ethyl)-N-methyl-amino]-1-oxo-2-buten-1-yl}amino)-7-cyclopentyloxy-quinazoline,
4-[(3-chloro-4-fluorophenyl)amino]-6-{[4-(N-cyclopropyl-N-methyl-amino)-1-oxo-2-buten-2-yl]amino}-7-cyclopentyloxy-quinazoline,
4-[(3-chloro-4-fluorophenyl)amino]-6-{[4-(N,N-dimethylamino)-1-oxo-2-buten-1-yl]amino }-7-[(R)-(tetrahydrofuran-2-yl)methoxy]-quinazoline,
4-[(3-chloro-4-fluorophenyl)amino]-6-{[4-(N,N-dimethylamino)-1-oxo-2-buten-1-yl]amino}-7-[(S)-(tetrahydrofuran-2-yl)methoxy]-quinazoline, 4-[(3-chloro-4-fluoro-phenyl)amino]-6-[(4-dimethylamino-cydohexyl)amino]-pyrimido[5,4-denotes]pyrimidine and
4-[(3-chloro-4-fluorophenyl)amino]-6-[3-(morpholin-4-yl)-propyloxy]-7-methoxy-quinazoline, optionally in the form of the physiologically acceptable acid addition salts thereof.

7. Pharmaceutical composition according to one of claims 1 to 6, **characterised in that** 2 is selected from the group consisting of
4-[(3-chloro-4-fluoro-phenyl)amino]-7-[4-((R)-6-methyl-2-oxo-morpholin-4-yl)-butyloxy]-6-((vinylcarbonyl)amino)-quinazoline,
4-[(3-chloro-4-fluoro-phenyl)amino]-7-(4-((S)-6-methyl-2-oxo-morpholin-4-yl)-butyloxy]-6-[(vinylcarbonyl)amino]-quinazoline,
4-[(3-chloro-4-fluoro-phenyl)amino]-7-(2-{4-[(*S*)-(2-oxo-tetrahydrofuran-5-yl)carbonyl]-piperazin-1-yl}-ethoxy)-6-[(vinylcarbonyl)amino]-quinazoline,
4-[(3-chloro-4-fluoro-phenyl)amino]-7-[2-((S)-6-methyl-2-oxo-morpholin-4-yl)-ethoxy]-6-[(vinylcarbonyl)amino]-quinazoline,
4-[(3-chloro-4-fluorophenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-quinazoline,
4-[(R)-(1-phenyl-ethyl)amino]-6-{[4-(morpholin-4-yl)-1-oxo-2-buten-1-yl]amino}-7-cyclopropylmethoxy-quinazoline and
4-((3-chloro-4-fluorophenyl)amino]-6-[3-(morpholin-4-yl)-propyloxy]-7-methoxy-quinazoline,
optionally in the form of the physiologically acceptable acid addition salts thereof.

8. Pharmaceutical composition according to one of claims 1 to 7, **characterised in that** it is in the form of a formulation suitable for inhalation.

9. Pharmaceutical composition according to claim 8, **characterised in that** it is a formulation selected from among inhalable powders, propellant-containing metered-dose aerosols and propellant-free inhalable solutions or suspensions.

10. Pharmaceutical composition according to claim 9, **characterised in that** it is an inhalable powder which contains **1** and **2** in admixture with suitable physiologically acceptable excipients selected from among the monosaccharides, disaccharides, oligo- and polysaccharides, polyalcohols, salts, or mixtures of these excipients.

11. Inhalable powder according to claim 9, **characterised in that** it is an inhalable powder which contains only active substances **1** and **2** as its ingredients.

12. Pharmaceutical composition according to claim 9, **characterised in that** it is a propellant-containing inhalable aerosol which contains **1** and **2** in dissolved or dispersed form.

13. Pharmaceutical composition according to claim 12, **characterised in that** it contains, as propellant gas, hydrocarbons such as n-propane, n-butane or isobutane or halohydrocarbons such as chlorinated and/or fluorinated derivatives of methane, ethane, propane, butane, cyclopropane or cyclobutane.

14. Pharmaceutical composition according to claim 13, **characterised in that** the propellant gas is TG11, TG12, TG134a, TG227 or mixtures thereof, preferably TG134a, TG227 or a mixture thereof.

15. Pharmaceutical composition according to claim 9, **characterised in that** it is a propellant-free inhalable solution or suspension which contains water, ethanol or a mixture of water and ethanol as solvent.

16. Pharmaceutical composition according to claim 15, **characterised in that** the pH is 2 - 7, preferably 2 -5.

17. Use of a composition according to one of claims 1 to 16 for preparing a medicament for treating inflammatory and/or obstructive diseases of the respiratory tract.

## Revendications

1. Médicament **caractérisé par** une teneur en un ou plusieurs anticholinergiques de **formule 1** 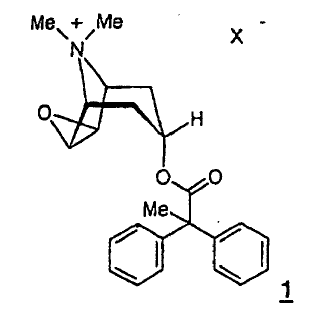
où
X⁻ représente un anion à une seule charge négative, de préférence un anion choisi dans le groupe consistant en chlorure, bromure, iodure, sulfate, phosphate, méthanesulfonate, nitrate, maléate, acétate, citrate, fumarate, tartrate, oxalate, succinate, benzoate et p-toluènesulfonate,
en combinaison avec un ou plusieurs inhibiteurs de EGFR-kinase **(2),** éventuellement sous forme de leurs énantiomères, de mélanges des énantiomères ou sous forme des racémates, éventuellement sous forme des solvates ou des hydrates ainsi éventuellement que conjointement avec un adjuvant pharmaceutiquement acceptable.

2. Médicament selon la revendication 1 **caractérisé en ce que** les principes actifs **1** et **2** sont contenus soit ensemble dans une seule forme d'administration soit dans deux formes d'administration séparées.

3. Médicament selon l'une des revendications 1 ou 2 **caractérisé en ce que**, dans les composés de formule **1,** X⁻ représente un anion à une seule charge négative choisi dans le groupe consistant en chlorure, bromure, p-toluènesulfonate et méthanesulfonate.

4. Médicament selon l'une des revendications 1 à 3 **caractérisé en ce que**, dans les composés de formule **1,** X⁻ représente bromure.

5. Médicament selon l'une des revendications 1 à 4 **caractérisé en ce que** 2 est choisi dans le groupe consistant en
4-[(3-chloro-4-fluoro-phényl)amino]-7-(2-{4-[(*S*)-(2-oxo-tétrahydrofuran-5-yl)-carbonyl]-pipérazin-1-yl}-éthoxy)-6-[(vinylcarbonyl)amino]-quinazoline,
4-[(3-chloro-4-fluoro-phényl)amino]-7-[2-((*S*)-6-méthyl-2-oxo-morpholin-4-yl)-éthoxy]-6-[(vinyl-carbonyl)amino]-quinazoline,
4-[(3-chloro-4-fluoro-phényl)amino]-7-[4-((R)-6-méthyl-2-oxo-morpolin-4-yl)-butyloxy]-6-[(vinyl-carbonyl)amino]-quinazoline,
4-[(3-chloro-4-fluoro-phényl)amino]-7-[4-((S)-6-méthyl-2-oxo-morpholin-4-yl)-butyloxy]-6-[(vinyl-carbonyl)amino]-quinazoline ,
4-[(3-chloro-4-fluorophényl)amino]-6- {[4-(morpholin-4-yl)-1-oxo-2-butén-1-yl]-amino}-7-cyclopropylméthoxy-quinazoline,
4-[(3-chloro-4-fluorophényl)amino]-6-{[4-(N,N-diéthylamino)-1-oxo-2-butén-1-yl]amino)-7-cyclo-propylméthoxy-quinazoline,
4-[(3-chloro-4-fluorophényl)amino]-6- {[4-(N,N-diméthylamino)-1-oxo-2-butén-1-yl]amino}-7-cydopropylméthoxy-quinazoline,
4-[(3-ehloro-4-fluorophényl)amino]-6-[(4- {N-[2-(éthoxycarbonyl)-éthyl]-N-[(éthoxycarbonyl)-méthyl]amino}-1-oxo-2-butén-1-yl)amino]-7-cyclopropyl-méthoxy-quinazoline,
4-[(R)-(1-phényl-éthyl)amino]-6-{[4-(morpholin-4-yl)-1-oxo-2-butén-1-yl]amino}-7-cyclopropyl-méthoxy-quinazoline,
4-[(*R*)-(1-phényl-éthyl)amino]-6-{[4-(morpholin-4-yl)-1-oxo-2-butén-1-yl]amino} -7-cyclopentyloxy-quinazoline,
4-[(3-chloro-4-fluoro-phényl)amino]-6- {[4-((*R*)-6-méthyl-2-oxo-morpholin-4-yl)-1-oxo-2-butén-1-yl]amino}-7-cyclopropylméthoxy-quinazoline,
4-[(3-chloro-4-fluorophényl)amino]-6- {[4-((*R*)-6-méthyl-2-oxo-morpholin-4-yl)-1-oxo-2-butén-1-yl]amino}-7-[(*S*)-(tétrahydrofuran-3-yl)oxy]-quinazoline,
4-[(3-chloro-4-fluoro-phényl)amino]-6-{[4-((R)-2-méthoxyméthyl-6-oxo-morpholin-4-yl)-1-oxo-2-butén-1-yl]amino}-7-cydopropylméthoxy-quinazoline,
4-[(3-chloro-4-fluoro-phényl)amino]-6-[2-((*S*)-6-méthyl-2-oxo-morpholin-4-yl)-éthoxy]-7-méthoxy-quinazoline,
4-[(3-chloro-4-fluoro-phényl)amino]-6-({4-[N-(2-méthoxy-éthyl)-N-méthyl-amino]-1-oxo-2-butén-1-yl} amino)-7-cyclopropylméthoxy-quinazoline,
4-[(3-chloro-4-fluorophényl)amino]-6- {[4-(N,N-diméthylamjno)-1-oxo-2-butén-1-yl]amino}-7-cyclopentyloxy-quinazoline,
4-[(*R*)-(1-phényl-éthyl)amino]-6-{[4-N,N-bis-(2-méthoxy-éthyl)-amino)-1-oxo-2-butén-1-yl]amino} -7-cyclopropyhnéthoxy-quinazoline,
4-[(*R*)-(1-phényl-éthyl)amino]-6-({4-[N-(2-méthoxy-éthyl)-N-éthyl-amino]-1-oxo-2-butén-1-yl} amino)-7-cyclopropylméthoxy-quinazoline,
4-[(*R*)-(1-phényl-éthyl)amino]-6-({4-[N-(2-méthoxy-éthyl)-N-méthyl-amino]-1-oxo-2-butén-1-yl}-amino)-7-cyclopropylméthoxy-quinazoline,
4-[(*R*)-(1-phényl-éthyl)amino]-6-({4-[N-(tétrahydropyran-4-yl)-N-méthyl-amino]-1-oxo-2-butén-1-yl}-amino)-7-cyclopropylméthoxy-quinazoline,
4-[(3-chloro-4-fluorophényl)amino]-6- {[4-(N,N-diméthylamino)-1-oxo-2-butén-1-yl]- amino)-7-((*R*)-tétrahydrofuran-3-yloxy)-quinazoline,
4-[(3-chloro-4-fluorophényl)amino]-6- ([4-(N,N-diméthylamino)-1-oxo-2-butén-1-yl]- amino)-7-((*S*)-tétrahydrofuran-3-yloxy)-quinazoline,
4-[(3-chloro-4-fluorophényl)amino]-6-({4-[N-(2-méthoxy-éthyl)-N-méthylamino]-1-oxo-2-butén-1-yl} amino)-7-cyclopentyloxy-quinazoline,
4-[(3-chloro-4-fluorophényl)amino]-6- {[4-[N-cyclopropyl-N-méthylamino]-1-oxo-2-butén-1-yl]-amino}-7-cyclopentyloxy-quinazoline,
4-[(3-chloro-4-fluorophényl)amino]-6- {[4-(N,N-diméthylamino)-1-oxo-2-butén-1-yl]- amino)-7-((*R*)-tétrahydrofuran-2-yl)méthoxy]-quinazoline,
4-[(3-chloro-4-fluorophényl)amino]-6- {[4-(N,N-diméthylamino)-1-oxo-2-butén-1-yl]- amino)-7-((S)-tétrahydrofuran-2-yl)méthoxy]-quinazoline,
4-[(3-chloro-4-fluorophényl)amino]-6-[3-(morpholin-4-yl)-propyloxy]-7-cyclopropyl-méthoxy-quinazoline,
4-[(3-éthynyl-phényl)amino]-6,7-bis-(2-méthoxy-éthoxy)-quinazoline,
4-[(3-chloro-4-fluorophényl)amino]-7-[3-(morpholin-4-yl)-propyloxy]-6-[(vinyl-carbonyl)amino]- quinazoline,
4-[(*R*)-(1-phényl-éthyl)amino]-6-(4-hydroxy-phényl)-7H-pyrrolo[2,3-d]pyrimidine,
3-cyano-4-[(3-chloro-4-fluorophényl)amino]-6- {[4-(N,N-diméthylamino)-1-oxo-2-butén-1-yl]amino}-7-éthoxy-quinoléine,
4- {[3-chloro-4-(3-fluoro-benzyloxy)-phényl]amino} -6-(5- {[(2-méthanesulfonyl-éthyl)amino]méthyl}-furan-2-yl)quinazoline, Cetuximab, Trastuzumab, ABX-EGF et Mab ICR-62, éventuellement sous forme de leurs sels d'addition d'acide physiologiquement acceptables.

6. Médicament selon l'une des revendications 1 à 5 **caractérisé en ce que** 2 est choisi dans le groupe consistant en
4-[(3-chloro-4-fluoro-phényl)amino]-7-(2-{4-[{(*S*)-(2-oxo-tétrahydrofuran-5-yl)-carbonyl]-pipérazin-1-yl} -éthoxy)-6-[(vinylcarbonyl)amino]-quinazoline,
4-[(3-chloro-4-fluoro-phényl)amino]-7-[2-((*S*)-6-méthyl-2-oxo-morpholin-4-yl)-éthoxy]-6-[(vinylcarbonyl)amino]-quinazoline,
4-[(3-chloro-4-fluoro-phényl)amino]-7-[4-((*R*)-6-méthyl-2-oxo-morpolin-4-yl)-butyloxy]-6-[(vinylcarbonyl)amino]-quinazoline,
4-[(3-chloro-4-fluoro-phényl)amino]-7-[4-((*S*)-6-méthyl-2-oxo-morpholin-4-yl)-butyloxy]-6-[(vinylcarbonyl)amino]-quinazoline ,
4-[(3-chloro-4-fluoro-phényl)amino]-7-[4-(2,2-diméthyl-6-oxo-morpholin-4-yl)-butyloxy]-6-[(vinylcarbonyl)amino]-quinazoline,
4-[(3-chloro-4-fluorophényl)amino]-6- {[4-(morpholin-4-yl)-1 -oxo-2-butén-1-yl]-amino}-7-cyclopropylméthoxy-quinazoline,
4-[(3-chloro-4-fluorophényl)amino]-6- {[4-(N,N-diéthylamino)-1-oxo-2-butén-1-yl]amino }-7-cyclopropylméthoxy-quinazoline,
4-[(3-chloro-4-fluorophényl)amino]-6- {[4-(N,N-diméthylamino)-1-oxo-2-butén-1-yl]amino}-7-cyclopropylméthoxy-quinazoline,
4-[(3-chloro-4-fluorophényl)amino]-6-[(4-{N-[2-(éthoxycarbonyl)-éthyl]-N-[(éthoxycarbonyl)méthyl]amino}-1-oxo-2-butén-1-yl)amino]-7-cyclopropylméthoxy-quinazoline,
4-[(*R*)-(1-phényl-éthyl)amino]-6- {[4-(morpholin-4-yl)-1-oxo-2-butén-1-yl]amino}-7-cyclopropylméthoxy-quinazoline,
4-[(*R*)-(1-phényl-éthyl)amino]-6- {[4-(morpholin-4-yl)-1-oxo-2-butén-1-yl]amino}-7-cyclopentyloxy-quinazoline,
4-[(3-chloro-4-fluoro-phényl)amino]-6- {[4-((*R*)-6-méthyl-2-oxo-morpholin-4-yl)-1-oxo-2-butén-1-yl]amino}-7-cyclopropylméthoxy-quinazoline,
4-[(3-chloro-4-fluoro-phényl)amino]-6-({4-[bis-(2-méthoxyéthyl)-amino]-1oxo-2-butén-1-yl]amino}-7-cyclopropylméthoxy-quinazoline,
4-[(3-chloro-4-fluorophényl)amino]-6-{[4-((*R*)-6-méthyl-2-oxo-morpholin-4-yl)-1-oxo-2-butén-1-yl]amino}-7-[(*S*)-(tétrahydrofuran-3-yl)oxy]-quinazoline,
4-[(3-chloro-4-fluoro-phényl)amino]-6- {[4-((*R*)-2-méthoxyméthyl-6-oxo-morpholin-4-yl)-1-oxo-2-butén-1-yl]amino} -7-cyclopropylméthoxy-quinazoline,
4-[(3-chloro-4-fluoro-phényl)amino]-6-[2-((S)-6-méthyl-2-oxo-morpholin-4-yl)-éthoxy]-7 -méthoxy-quinazoline,
4-[(3-chloro-4-fluorophényl)amino]-6-({4-[N-(2-méthoxy-éthyl)-N-méthyl-amino]-1-oxo-2-butén-1-yl}-amino)-7-cyclopropylméthoxy-quinazoline,
4-[(3-chloro-4-fluorophényl)amino]-6-{[4-(N,N-diméthylamjno)-1-oxo-2-butén-1-yl]amino} -7-cydopentyloxy-quinazoline,
4-[(3-chloro-4-fluorophényl)amino]-6-{[4-(S)-2-méthoxyméthyl-6-oxo-morpholin-4-yl)-1-oxo-2-butén-1-yl]amino}-7-cyclopropylméthoxy-quinazoline,
4-[(*R*)-(1-phényl-éthyl)amino]-6-{[4-(N,N-bis-(2-méthoxy-éthyl)-amino)-1-oxo-2-buten-1-yl]amino) -7-cyclopropylméthoxy-quinazoline,
4-[(*R*)-(1-phényl-éthyl)amino]-6-({4-[N-(2-méthoxy-éthyl)-N-éthyl-amino]-1-oxo-2-buten-1-yl}amino)-7-cyclopropylméthoxy-quinazoline,
4-[(*R*)-(1-phényl-éthyl)amino]-6-({4-[N-(2-méthoxy-éthyl)-N-méthyl-amino]-1-oxo-2-butén-1-yl} amino)-7-cyclopropylméthoxy-quinazoline,
4-[(R)-(1-phényl-éthyl)amino]-6-({4-[N-(tétrahydropyran-4-yl)-N-méthyl-amino]-1-oxo-2-butén-1-yl}amino)-7-cyclopropylméthoxy-quinazoline,
4-[(3-chloro-4-fluorophényl)amino]-6- {[4-(N,N-diméthylamino)-1-oxo-2-butén-1-yl]- amino)-7-((*R*)-tétrahydrofuran-3-yloxy)-quinazoline,
4-[(3-chloro-4-fluorophényl)amino]-6- {[4-(N,N-diméthylamino)-1-oxo-2-butén-1-yl]- amino)-7-((*S*)-tétrahydrofuran-3-yloxy)-quinazoline,
4-[(3-chloro-4-fluorophényl)amino]-6-({4-[N-(2-méthoxy-éthyl)-N-méthylamino]-1-oxo-2-butén-1-yl} amino)-7-cyclopentyloxy-quinazoline,
4-[(3-chloro-4-fluorophényl)amino]-6-{[4-[N-cyclopropyl-N-méthylamino]-1-oxo-2-butén-1-yl]amino} -7-cyclopentyloxy-quinazoline,
4-[(3-chloro-4-fluorophényl)amino]-6- {[4-(N,N-diméthylamino)-1-oxo-2-butén-1-yl]- amino)-7-((*R*)-tétrahydrofuran-2-yl)méthoxy]-quinazoline,
4-[(3-chloro-4-fluorophényl)amino]-6-{[4-(N,N-diméthylamino)-1-oxo-2-butén-1-yl]- amino)-7-((*S*)-tétrahydrofuran-2-yl)méthoxy]-quinazoline,
4-[(3-chloro-4-fluorophényl)amino]-6-[(4-diméthylamino-cyclohexyl)amino]-pyrimido[5,4-d]pyrimidine et
4-[(3-chloro-4-fluorophényl)amino]-6-[3-(morpholin-4-yl)-propyloxy]-7-méthoxyquinazoline, éventuellement sous forme de leurs sels d'addition d'acide physiologiquement acceptables.

7. Médicament selon l'une des revendications 1 à 6 **caractérisé en ce que** 2 est choisi dans le groupe consistant en
4-[(3-chloro-4-fluoro-phényl)amino]-7-[4-((*R*)-6-méthyl-2-oxo-morpolin-4-yl)-butyloxy]-6-[(vinylcarbonyl)amino]-quinazoline,
4-[(3-chloro-4-fluoro-phényl)amino]-7-[4-((*S*)-6-méthyl-2-oxo-morpholin-4-yl)-butyloxy]-6-[(vinylcarbonyl)amino]-quinazoline ,
4-[(3-chloro-4-fluoro-phényl)amino]-7-(2- {4-[(*S*)-(2-oxo-tétrahydrofuran-5-yl)-carbonyl]-pipérazin-1-yl}-éthoxy)-6-[(vinylcarbonyl)amino]-quinazoline,
4-[(3-chloro-4-fluoro-phényl)amino]-7-[2-((*S*)-6-méthyl-2-oxo-morpholin-4-yl)-éthoxy]-6-[(vinylcarbonyl)amino]-quinazoline,
4-[(3-chloro-4-fluorophényl)amino]-6-[(4- {N-[2-(éthoxycarbonyl)-éthyl]-N-[(éthoxycarbonyl)méthyl]amino}-1-oxo-2-butén-1-yl)amino]-7-cyclopropylméthoxy-quinazoline,
4-[(*R*)-(1-phényl-éthyl)amino]-6- {[4-(morpholin-4-yl)-1-oxo-2-butén-1-yl]amino}-7-cyclopropylméthoxy-quinazoline et
4-[(3-chloro-4-fl uorophényl)amino]-6-[3-(morpholin-4-yl)-propyloxy]-7-méthoxy-quinazoline, éventuellement sous forme de leurs sels d'addition d'acide physiologiquement acceptables.

8. Médicament selon l'une des revendications 1 à 7 **caractérisé en ce qu'**il est sous forme d'une forme d'administration appropriée à l'inhalation.

9. Médicament selon la revendication 8 **caractérisé en ce qu'**il s'agit d'une forme d'administration choisie dans le groupe poudre pour inhalation, aérosols de dosage contenant un gaz propulseur et solutions ou suspensions pour inhalation sans gaz propulseur.

10. Médicament selon la revendication 9 **caractérisé en ce que** c'est une poudre pour inhalation qui contient **1** et **2** en mélange avec des adjuvants physiologiquement acceptables appropriés choisis dans le groupe consistant en les monosaccharides, les disaccharides, les oligo- et polysaccharides, les polyalcools, les sels ou des mélanges de ces adjuvants.

11. Médicament selon la revendication 9 **caractérisé en ce que** c'est une poudre pour inhalation qui contient comme constituants uniquement les principes actifs **1** et **2**.

12. Médicament selon la revendication 9 **caractérisé en ce qu'**il s'agit d'un aérosol pour inhalation contenant un gaz propulseur qui contient **1** et **2** sous forme dissoute ou dispersée.

13. Médicament selon la revendication 12 **caractérisé en ce qu'**il contient comme gaz propulseur des hydrocarbures comme le n-propane, le n-butane ou l'isobutane ou des halogénohydrocarbures comme des dérivés chlorés et/ou fluorés du méthane, de l'éthane, du propane, du butane, du cyclopropane ou du cyclobutane.

14. Médicament selon la revendication 13 **caractérisé en ce que** le gaz propulseur est TG11, TG12, TG134a, TG227 ou des mélanges de ceux-ci, de préférence TG134a, TG227 ou un mélange de ceux-ci.

15. Médicament selon la revendication 9 **caractérisé en ce qu'**il s'agit d'une solution ou suspension pour inhalation sans gaz propulseur qui contient comme solvant de l'eau, de l'éthanol ou un mélange d'eau et d'éthanol.

16. Médicament selon la revendication 15 **caractérisé en ce que** le pH est 2-7, de préférence 2-5.

17. Utilisation d'une composition selon l'une des revendications 1 à 16 pour la production d'un médicament pour le traitement des maladies inflammatoires et/ou obstructives des voies respiratoires.
